# EUROPÄISCHE PATENTSCHRIFT

(11) **EP 1 893 587 B1**
(45) Veröffentlichungstag und Bekanntmachung des Hinweises auf die Patenterteilung: **25.01.2012**
(21) Anmeldenummer: 06743106.4
(22) Anmeldetag: 02.06.2006
(51) Int. Cl.: C07D 239/78, C07D 239/80, C07D 239/84

(54) **VERFAHREN ZUR HERSTELLUNG VON DIHYDROCHINAZOLINEN**
METHOD FOR PRODUCING DIHYDROQUINAZOLINES
PROCEDE POUR PRODUIRE DES DIHYDROQUINAZOLINES

(30) Priorität: 15.06.2005 DE 102005027517
(43) Veröffentlichungstag der Anmeldung: 05.03.2008
(73) Patentinhaber: AiCuris GmbH & Co. KG, 42117 Wuppertal (DE)
(72) Erfinder: GOOSSEN, Käthe, 67663 Kaiserslautern (DE); KUHN, Oliver, 6581 Rosport (LU); BERWE, Mathias, 45549 Sprockhövel (DE); KRÜGER, Joachim, 40489 Düsseldorf (DE); MILITZER, Hans-Christian, 51519 Odenthal (DE)
(74) Vertreter: Scheuermann, Erik
(86) Internationale Anmeldenummer: PCT/EP2006/005298
(87) Internationale Veröffentlichungsnummer: WO 2006/133822

(56) Entgegenhaltungen:
- WO-A-2004/072048
- WO-A-2004/096778
- XIN Z ET AL: "A practical and efficient intramolecular Michael addition of ureas to alpha,beta-unsaturated esters" TETRAHEDRON LETTERS, ELSEVIER, AMSTERDAM, NL, Bd. 41, Nr. 8, Februar 2000 (2000-02), Seiten 1147-1150, XP004188577 ISSN: 0040-4039
- MOLINA ET AL.: "Tetrabutylammonium Fluoride Promoted Intramolecular Nucleophilic Attack of a Carbodiimide group on an alpha, beta-Unsaturated Ester Group" SYNTHESIS, Nr. 3, 1998, Seiten 283-287, XP002394931

## Beschreibung

Die vorliegende Erfindung betrifft ein Verfahren zur Herstellung von Dihydrochinazolinen, die zur Herstellung von Arzneimitteln dienen.

Die nach dem erfindungsgemäßen Verfahren hergestellten Verbindungen eignen sich zur Verwendung als antivirale Mittel, insbesondere gegen Cytomegaloviren, wie in WO 04/072048 und WO 04/096778 beschrieben. Eine Synthese von Dihydrochinazolinen wird in Xin, Z. et al., THL 41 (2000) 1147-1150 und Molina, P. et al., Synthesis, 3 (1998) 283-287 beschrieben.

Die dort beschriebene Synthese der Dihydrochinazoline erfolgt ausgehend von einem 2-halogensubstituierten Anilin (A), das mittels Heck-Kupplung in ein 2-Aminozimtsäurederivat (B) überführt wird. Durch Umsetzung mit Triphenylphosphin in Tetrachlorkohlenstoff wird ein Phosphinimid (C) hergestellt, das anschließend mit einem Isocyanat unter Freisetzung von Triphenylphosphinoxid zu einem Carbodiimid (D) umgesetzt wird. Durch Reaktion des Carbodiimids (D) mit einem Amin entsteht der Dihydrochinazolin-Methylester (E), der durch Chromatographie an einer chiralen Phase in die Enantiomere getrennt wird. Anschließend erfolgt die Verseifung zur Dihydrochinazolin-Säure (F1) unter Standardbedingungen. Die folgenden Schemata 1 und 2 veranschaulichen die Synthese.

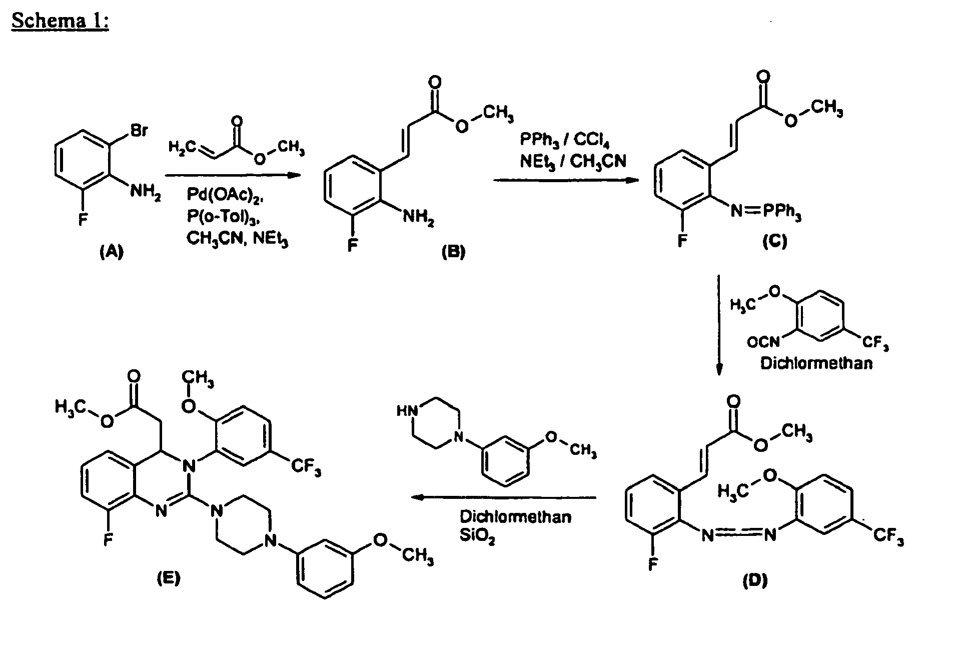

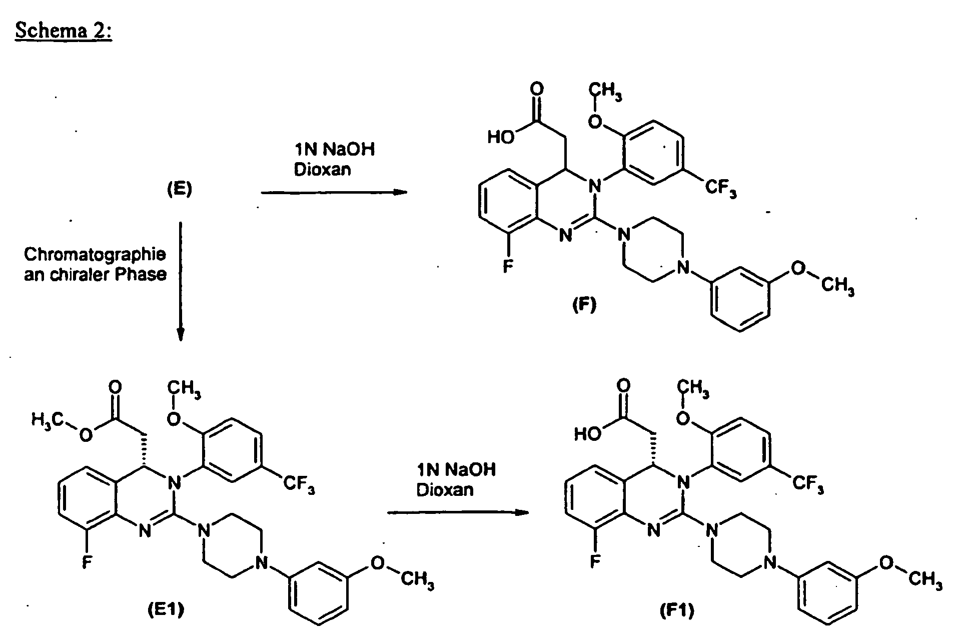

Die oben beschriebenen Reaktionsschritte bergen bei der Durchführung im technischen Maßstab deutliche Risiken, und es entstehen Nebenprodukte, sowie stöchiometrische Mengen organischer Abfallprodukte. Mit dem Phosphinimid (C) und dem Carbodiimid (D) entstehen während der Reaktionssequenz Zwischenstufen mit hochreaktiven Funktionalitäten, die in erheblichem Maße zu Nebenprodukten führen. Die Nebenprodukte sind nur durch sehr aufwändige chromatographische Reinigung oder durch ein aufwändiges Extraktionsverfahren abzutrennen.

Weiterhin entsteht bei der Umsetzung der Verbindung der Formel (C) zur Verbindung der Formel (D) Triphenylphosphinoxid in stöchiometrischen Mengen, das in einem aufwändigen Verfahren chromatographisch von dem gewünschten Produkt abgetrennt wird. Eine Chromatographie ist bei der Synthese von Verbindungen im technischen Maßstab besonders nachteilig, da sie zeit- und arbeitsaufwändig ist und größere Mengen an Lösungsmittel verbraucht.

Die Enantiomerentrennung der Verbindung der Formel (E) erfolgt in einem aufwändigen Verfahren durch Chromatographie an einer chiralen Phase, wobei das unerwünschte R-Enantiomer als Abfallprodukt anfällt.

Die Aufgabe der vorliegenden Erfindung ist es, ein technisch anwendbares Verfahren zur Herstellung eines Dihydrochinazolins der Formel (I), insbesondere von (*S*)-{8-Fluor-2-[4-(3-methoxyphenyl)piperazin-1-yl]-3-(2-methoxy-5-trifluormethylphenyl)-3,4-dihydrochinazolin-4-yl}essigsäure, bereitzustellen, bei welchem die Nachteile der aus dem Stand der Technik bekannten, vorstehenden Verfahrensschritte vermieden werden und bei dem kein unerwünschtes R-Enantiomer als Abfallprodukt anfällt.

Diese Aufgabe wird gemäß der vorliegenden Erfindung, wie folgt, gelöst. Die folgenden Schemata 3 und 4 veranschaulichen die einzelnen Reaktionsschritte.

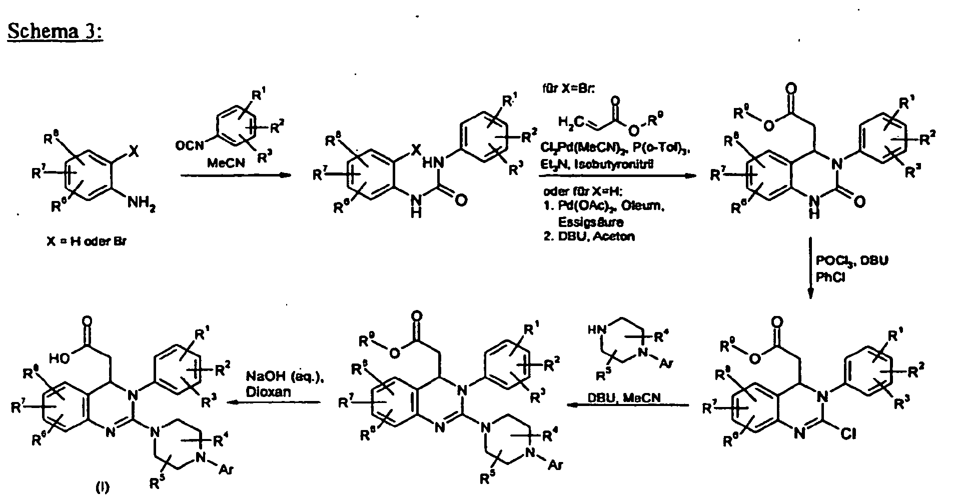

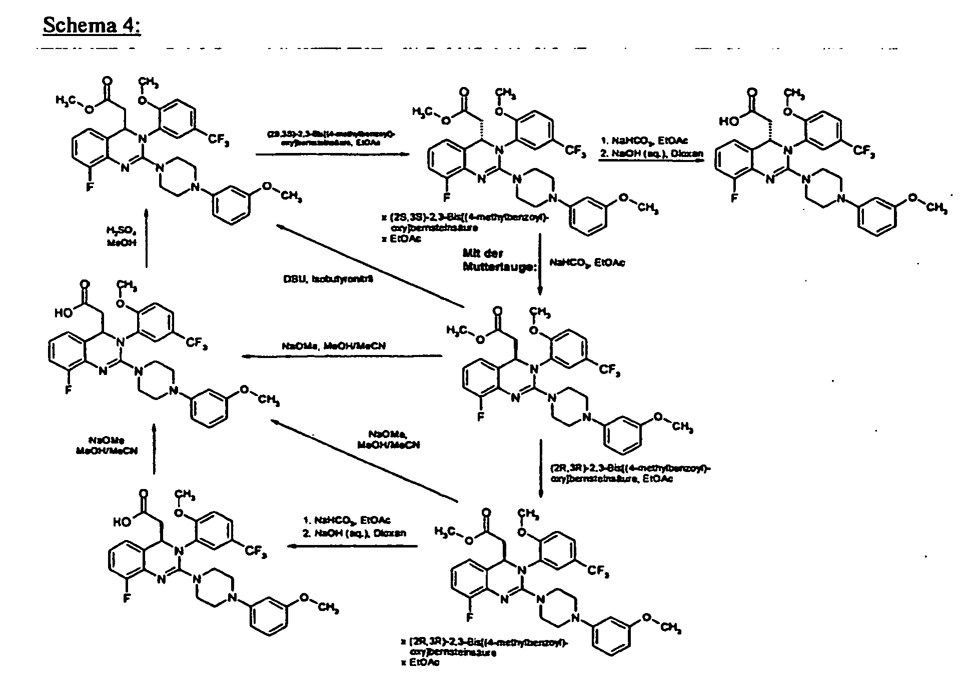

Überraschend wurde nun gefunden, dass Verbindungen der Formel (I) durch das erfindungsgemäße Verfahren, d. h. durch Umsetzung des 2-halogensubstituierten Anilins mit einem Isocyanat und einer anschließenden' Heckreaktion mit Acrylsäurealkylester, bevorzugt Acrylsäuremethylester, hergestellt werden können und dass damit ein Phosphinimid und ein Carbodiimid als reaktive Zwischenstufe bzw. die Entstehung stöchiometrischer Mengen Triphenylphosphinoxid vermieden werden kann.

Des weiteren wurde überraschend gefunden, dass die Verbindungen der Zwischenstufen Kristalle bilden und sich durch Kristallisation ohne Chromatographie oder Extraktion reinigen lassen, wodurch die technische Anwendung dieser Verfahrensstufen ermöglicht wird.

Des weiteren wurde überraschend gefunden, dass sich der Baustein {8-Fluor-3-[2-methoxy-5-(trifluormethyl)phenyl]-2-oxo-1,2,3,4-tetrahydrochinazolin-4-yl}essigsäuremethylester effizient über eine ortho-Palladierung synthetisieren lässt. Hierbei wird N-(2-Fluorphenyl)-N'-[2-methoxy-5-(trifluormethyl)phenyl]harnstoff mit Methylacrylat und einem Oxidationsmittel in Gegenwart einer Säure zu Methyl(2E)-3-{3-fluor-2-[({[2-methoxy-5-(trifluormethyl)phenyl]amino}carbonyl)-amino]phenyl}acrylat umgesetzt. Danach erfolgt der Ringschluss zum Tetrahydrochinazolin unter basischen Reaktionsbedingungen.

Des weiteren wurde überraschend gefunden, dass sich die {8-Fluor-2-[4-(3-methoxyphenyl)-piperazin-1-yl]-3-[2-methoxy-5-(trifluonmethyl)phenyl]-3,4-dihydrochinazolin-4-yl}essigsäure-alkylester, bevorzugt der {8-Fluor-2-[4-(3-methoxyphenyl)-piperazin-1-yl]-3-[2-methoxy-5-(trifluormethyl)phenyl]-3,4-dihydrochinazolin-4-yl}essigsäuremethylester, durch Kristallisation mit (2*S*,3*S*)-2,3-Bis[(4-methylbenzoyl)oxy]bernsteinsäure in die Enantiomere trennen lassen.

Des weiteren wurde überraschend gefunden, dass sich das R-Enantiomer der {8-Fluor-2-[4-(3-methoxyphenyl)-piperazin-1-yl]-3-[2-methoxy-5-(trifluormethyl)phenyl]-3,4-dihydrochinazolin-4-yl}essigsäurealkylester, bevorzugt des {8-Fluor-2-[4-(3-methoxyphenyl)-piperazin-1-yl]-3-[2-methoxy-5-(trifluormethyl)phenyl]-3,4-dihydrochinazolin-4-yl}essigsäuremethylesters, nach der Verseifung des Alkyl- bzw. Methylesters auf der Stufe der Säure basisch racemisieren lassen und sich nach erneuter Veresterung durch Kristallisation mit (2*S*,3*S*)-2,3-Bis[(4-methylbenzoyl)-oxy]bernsteinsäure trennen lassen, wodurch die Gesamt-Ausbeute an S-Enantiomer erhöht wird.

Im einzelnen umfasst das erfindungsgemäße Verfahren zur Herstellung einer Verbindung der Formel (I) 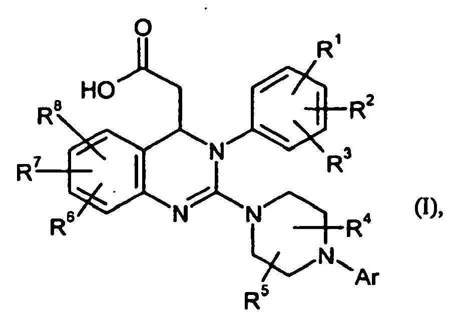 in welcher
- Ar: für Aryl steht, worin Aryl substituiert sein kann mit 1 bis 3 Substituenten, wobei die Substituenten unabhängig voneinander ausgewählt werden aus der Gruppe, bestehend aus Alkyl, Alkoxy, Formyl, Carboxyl, Alkylcarbonyl, Alkoxycarbonyl, Trifluormethyl, Halogen, Cyano, Hydroxy, Amino, Alkylamino, Aminocarbonyl und Nitro,
worin Alkyl substituiert sein kann mit 1 bis 3 Substituenten, wobei die Substituenten unabhängig voneinander ausgewählt werden aus der Gruppe, bestehend aus Halogen, Amino, Alkylamino, Hydroxy und Aryl,
oder zwei der Substituenten am Aryl zusammen mit den Kohlenstoffatomen, an die sie gebunden sind, ein 1,3-Dioxolan, einen Cyclopehtan-Ring oder einen Cyclohexan-Ring bilden und ein gegebenenfalls vorhandener dritter Substituent unabhängig davon aus der genannten Gruppe ausgewählt wird,
- R¹: für Wasserstoff, Amino, Alkyl, Alkoxy, Alkylamino, Alkylthio, Cyano, Halogen, Nitro oder Trifluormethyl steht,
- R²: für Wasserstoff, Alkyl, Alkoxy, Alkylthio, Cyano, Halogen, Nitro oder Trifluormethyl steht,
- R³: für Amino, Alkyl, Alkoxy, Alkylamino, Alkylthio, Cyano, Halogen, Nitro, Trifluormethyl, Alkylsulfonyl oder Alkylaminosulfonyl steht
oder
einer der Reste R¹, R² und R³ für Wasserstoff, Alkyl, Alkoxy, Cyano, Halogen, Nitro oder Trifluormethyl steht und die anderen beiden zusammen mit den Kohlenstoffatomen, an die sie gebunden sind, ein 1,3-Dioxolan, einen Cyclopentan-Ring oder einen Cyclohexan-Ring bilden,
- R⁴: für Wasserstoff oder Alkyl steht,
- R⁵: für Wasserstoff oder Alkyl steht
oder
die Reste R⁴ und R⁵ im Piperazin-Ring an genau gegenüberliegenden Kohlenstoffatomen gebunden sind und eine gegebenenfalls mit 1 bis 2 Methylgruppen substituierte Methylen-Brücke bilden,
- R⁶: für Wasserstoff, Alkyl, Alkoxy, Alkylthio, Formyl, Carboxyl, Aminocarbonyl, Alkylcarbonyl, Alkoxycarbonyl, Trifluormethyl, Halogen, Cyano, Hydroxy oder Nitro steht,
- R⁷: für Wasserstoff, Alkyl, Alkoxy, Alkylthio, Formyl, Carboxyl, Alkylcarbonyl, Alkoxycarbonyl, Trifluormethyl, Halogen, Cyano, Hydroxy oder Nitro steht
und
- R⁸: für Wasserstoff, Alkyl, Alkoxy, Alkylthio, Formyl, Carboxyl, Alkylcarbonyl, Alkoxycarbonyl, Trifluormethyl, Halogen, Cyano, Hydroxy oder Nitro steht,
die Verseifung des Esters einer Verbindung der Formel (II) 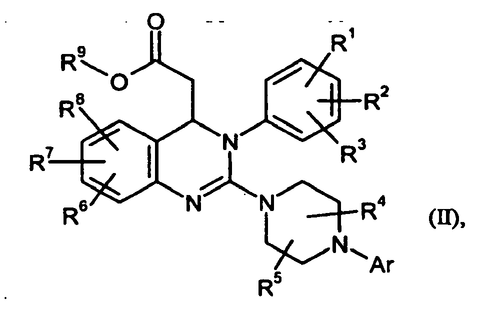 in welcher
- Ar, R¹, R²,R³, R⁴, R⁵, R⁶, R⁷ und R⁸: die oben angegebene Bedeutung haben, und
- R⁹: für C₁-C₄-Alkyl steht,
mit einer Base oder einer Säure.

Die Verbindung der Formel (II) kann hergestellt werden durch Umsetzung einer Verbindung der Formel (III) 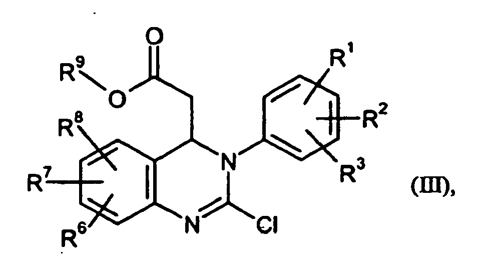 in welcher
- R¹, R²,R³, R⁶, R⁷ und R⁸: die oben angegebene Bedeutung haben, und
- R⁹: für C₁-C₄-Alkyl steht,
in Gegenwart einer Base
mit einer Verbindung der Formel (IV) 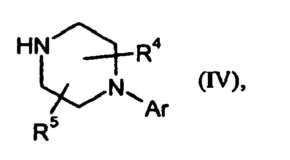 in welcher
- Ar, R⁴ und R⁵: die oben angegebene Bedeutung haben.

Die Verbindung der Formel (III) kann hergestellt werden durch Umsetzung einer Verbindung der Formel (V) 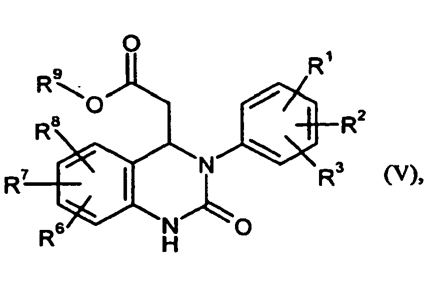 in welcher
- R¹, R²,R³, R⁶, R⁷ und R⁸: die oben angegebene Bedeutung haben, und
- R⁹: für C₁-C₄-Alkyl steht,
mit Phosphoroxychlorid, Phosphortrichlorid oder Phosphorpentachlorid in Gegenwart einer Base.

Die Verbindung der Formel (V) kann hergestellt werden durch Umsetzung einer Verbindung der Formel (VI) 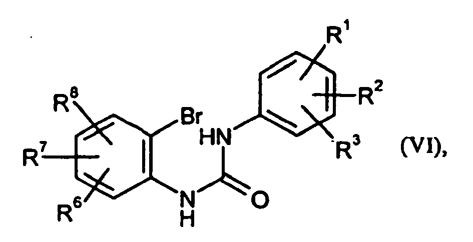 in welcher
- R¹, R², R³, R⁶, R⁷ und R⁸: die oben angegebene Bedeutung haben,
mit einer Verbindung der Formel 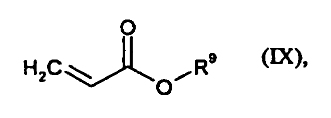 in welcher
- R⁹: für C₁-C₄-Alkyl steht,
in Gegenwart eines Palladiumkatalysators und einer Base.

Verbindungen der Formeln (IV), (VI) und (IX) sind dem Fachmann an sich bekannt oder lassen sich nach üblichen, literaturbekannten Verfahren herstellen.

In einem alternativen Verfahren kann eine Verbindung der Formel (V) hergestellt werden, indem eine Verbindung der Formel (VII) 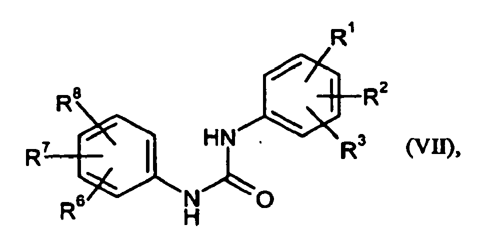 in welcher
- R¹, R², R³, R⁶, R⁷ und R⁸: die oben angegebene Bedeutung haben,
in der ersten Stufe mit einer Verbindung der Formel (IX) in Essigsäure in Gegenwart eines Palladiumkatalysators, eines Oxidationsmittels und einer Säure zu einer Verbindung der Formel (VIII) 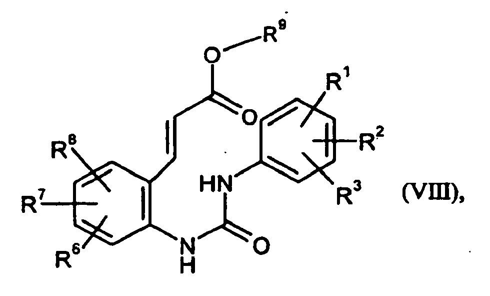 in welcher
- R¹, R², R³, R⁶, R⁷ und R⁸: die oben angegebene Bedeutung haben, und
- R⁹: für C₁-C₄-Alkyl steht,
und in der zweiten Stufe mit einer Base zu einer Verbindung der Formel (V) umgesetzt wird.

Verbindungen der Formel (VII) sind dem Fachmann an sich bekannt oder lassen sich nach üblichen, literaturbekannten Verfahren herstellen.

Gemäß einer bevorzugten Ausführungsform der vorliegenden Erfindung steht in den Syntheseverfahren der Rest R⁹ in den Verbindungen der Formeln (I), (III), (V), (VIII) und (IX) für Methyl.

Des weiteren umfasst die vorliegende Erfindung Verbindungen der Formel (III) 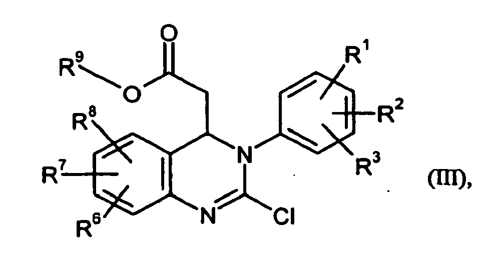 in welcher
- R¹, R², R³, R⁶, R⁷ und R⁸: die oben angegebene Bedeutung haben, und
- R⁹: für C₁-C₄-Alkyl steht.

Bevorzugt sind Verbindungen der Formel (III) in welcher R⁹ für Methyl steht.

Des weiteren umfasst die vorliegende Erfindung Verbindungen der Formel (V) 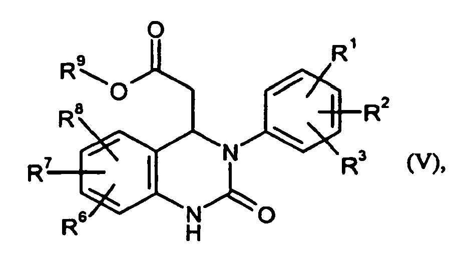 in welcher
- R¹, R², R³, R⁷ und R⁸: die oben angegebene Bedeutung haben, R⁶ fü Alkyl, Alkoxy, Alkylthio, Formyl, Carboxyl, Aminocarboxyl, Alkylcarbonyl, Alkoxycarbonyl, Trifluromethyl, Halogen, Cyano, Hydroxy oder Nitro steht, und
- R⁹: für C₁-C₄-Alkyl steht.

Bevorzugt sind Verbindungen der Formel (V) in welcher R⁹ für Methyl steht.

Gemäß einer besonders bevorzugten Ausführungsform der vorliegenden Erfindung ist die Verbindung der Formel (I) folgende:

### {8-Fluor-2-[4-(3-methoxyphenyl)-piperazin-1-yl]-3-[2-methoxy-5-(trifluormethyl)phenyl]-3,4-dihydrochinazolin-4-yl}essigsäure

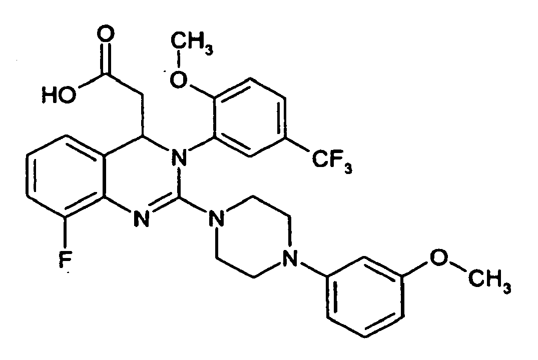

Gemäß einer besonders bevorzugten Ausführungsform der vorliegenden Erfindung ist die Verbindung der Formel (II) folgende:

### {8-Fluor-2-[4-(3-methoxyphenyl)-piperazin-1-yl]-3-[2-methoxy-5-(trifluormethyl)phenyl]-3,4-dihydrochinazolin-4-yl}essigsäuremethylester

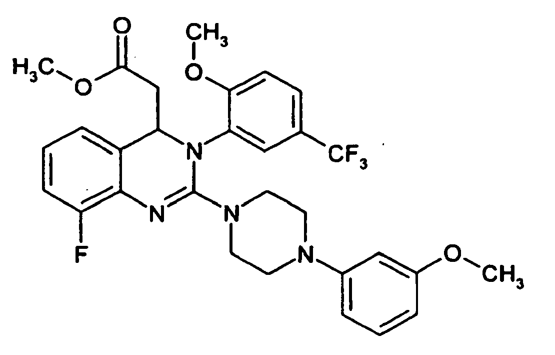

Gemäß einer besonders bevorzugten Ausführungsform der vorliegenden Erfindung ist die Verbindung der Formel (III) folgende:

### 2-Chlor-8-fluor-3-[2-methoxy-5-(trifluormethyl)phenyl]-3,4-dihydrochinazolin-4-yl}essigsäure-methylester

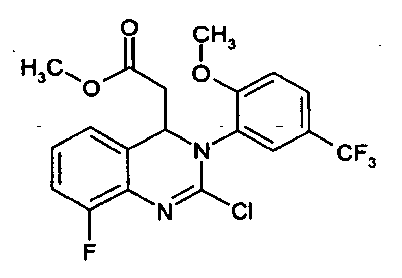

Gemäß einer besonders bevorzugten Ausführungsform der vorliegenden Erfindung ist die Verbindung der Formel (IV) folgende:

### 1-(3-Methoxyphenyl)piperazin

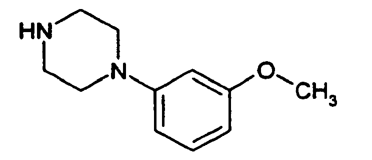

Gemäß einer besonders bevorzugten Ausführungsform der vorliegenden Erfindung ist die Verbindung der Formel (V) folgende:

### {8-Fluor-3-[2-methoxy-5-(trifluormethyl)phenyl]-2-oxo-1,2,3,4-tetrahydrochinazolin-4-yl }essigsäuremethylester

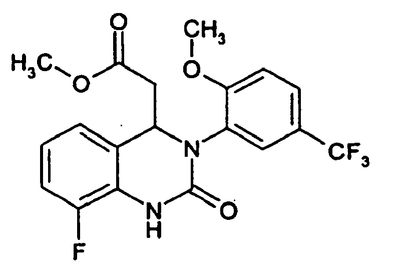

Gemäß einer besonders bevorzugten Ausführungsform der vorliegenden Erfindung ist die Verbindung der Formel (VI) folgende:

### N-(2-Brom-6-fluorphenyl)-N'-[2-methoxy-5-(trifluormethyl)phenyl]harnstoff

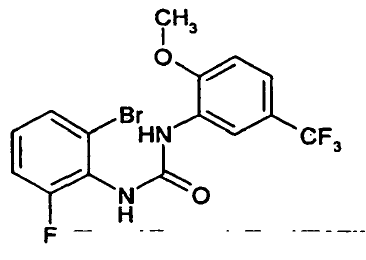

Gemäß einer besonders bevorzugten Ausführungsform der vorliegenden Erfindung ist die Verbindung der Formel (VII) folgende:

### N-(2-Fluorphenyl)-N'-[2-methoxy-5-(trifluormethyl)phenyl]harnstoff

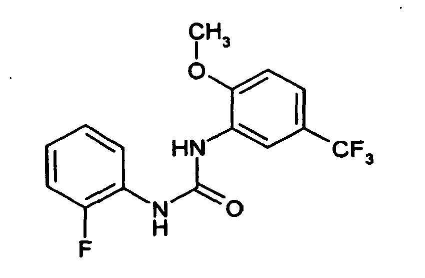

Gemäß einer besonders bevorzugten Ausführungsform der vorliegenden Erfindung ist die Verbindung der Formel (VIII) folgende:

### Methyl(2E)-3-{3-fluor-2-[({[2-methoxy-5-(trifluormethyl)phenyl]amino}carbonyl)amino]phenyl}-acrylat

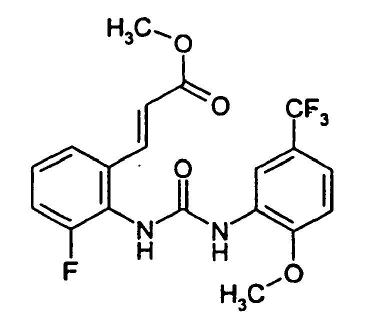

Gemäß einer besonders bevorzugten Ausführungsform der vorliegenden Erfindung ist die Verbindung der Formel (IX) folgende:

### Acrylsäuremethylester

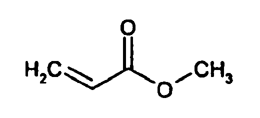

Die Verseifung des Esters einer Verbindung der Formel (II) zu einer Verbindung der Formel (I) erfolgt durch Umsetzung einer Verbindung der Formel (II) mit einer Base in einem inerten Lösungsmittel, in einem Temperaturbereich von 18°C bis zum Rückfluss des Lösungsmittels, bevorzugt bei 18 bis 50°C, besonders bevorzugt bei 20 bis 30°C, bei Normaldruck, innerhalb von beispielsweise 0,5 bis 10 Stunden, bevorzugt innerhalb von 1 bis 5 Stunden.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Alkoholate wie Natrium- oder Kaliummethanolat oder Natrium- oder Kaliumethanolat, wobei die Base gegebenenfalls in wässriger Lösung vorliegt.

Inerte Lösungsmittel sind beispielsweise Ether wie 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, oder Wasser, oder Gemische von Lösungsmitteln.

Bevorzugt ist Natriumhydroxid in Wasser und Dioxan.

Die Verseifung des Esters einer Verbindung der Formel (II) zu einer Verbindung der Formel (I) erfolgt durch Umsetzung einer Verbindung der Formel (II) mit einer Säure in einem Lösungsmittel in Gegenwart von Wasser, in einem Temperaturbereich von 18°C bis zum Rückfluss des Lösungsmittels, bevorzugt bei 18 bis 50°C, besonders bevorzugt bei 20 bis 30°C, bei Normaldruck, innerhalb von beispielsweise 0,5 bis 48 Stunden, bevorzugt innerhalb von 5 bis 24 Stunden.

Säuren in einem Lösungsmittel sind beispielsweise Salzsäure, Schwefelsäure oder Phosphorsäure in Dioxan oder Tetrahydrofuran.

Bevorzugt ist Salzsäure in Dioxan.

Die Synthese einer Verbindung der Formel (II) aus einer Verbindung der Formel (III) und einer Verbindung der Formel (IV) in Gegenwart einer Base erfolgt in einem inerten Lösungsmittel, in einem Temperaturbereich von 40°C bis zum Rückfluss des Lösungsmittels, bevorzugt bei Rückfluss des Lösungsmittels, bei Normaldruck, innerhalb von beispielsweise 5 bis 48 Stunden, bevorzugt innerhalb von 10 bis 24 Stunden.

Basen sind beispielsweise Amide wie Natriumamid, Lithium-bis-(trimethylsilyl)amid oder Lithiumdiisopropylamid, oder Aminbasen wie 1,8-Diazabicyclo[5.4.0]undec-7-en (DBU), 1-(3-Methoxyphenyl)piperazin oder Triethylamin, oder andere Basen wie Kalium-tert.-butylat oder Natriumhydrid.

Inerte Lösungsmittel sind beispielsweise Chlorbenzol oder Ether wie 1,2-Dimethoxyethan, Dioxan, Glykoldimethyl-ether oder Diethylenglykoldimethylether.

Bevorzugt ist DBU in Dioxan.

Die Umsetzung einer Verbindung der Formel (V) zu einer Verbindung der Formel (III) erfolgt durch Reaktion einer Verbindung der Formel (V) mit Phosphoroxychlorid, Phosphortrichlorid oder Phosphorpentachlorid, bevorzugt ist Phosphoroxychlorid, in Gegenwart einer Base in einem inerten Lösungsmittel, in einem Temperaturbereich von 40°C bis zum Rückfluss des Lösungsmittels, bevorzugt bei Rückfluss des Lösungsmittels, bei Normaldruck, innerhalb von beispielsweise 5 bis 48 Stunden, bevorzugt innerhalb von 10 bis 24 Stunden.

Basen sind beispielsweise Amine wie 1,8-Diazabicyclo[5.4.0]undec-7-en (DBU), Pyridin oder Triethylamin, oder Amide wie Natriumamid, Lithium-bis-(trimethylsilyl)amid oder Lithiumdiisopropylamid, oder andere Basen wie Kalium-tert.-butylat.

Inerte Lösungsmittel sind beispielsweise Kohlenwasserstoffe wie Benzol, Xylol, Toluol oder Chlorbenzol.

Bevorzugt ist DBU in Chlorbenzol.

Die Umsetzung einer Verbindung der Formel (VI) zu einer Verbindung der Formel (V) erfolgt durch Reaktion einer Verbindung der Formel (VI) mit einer Verbindung der Formel (IX) in Gegenwart eines Palladiumkatalysators und einer Base in einem inerten Lösungsmittel, in einem Temperaturbereich von 40°C bis zum Rückfluss des Lösungsmittels, bevorzugt bei Rückfluss des Lösungsmittels, bei Normaldruck, innerhalb von beispielsweise 5 bis 48 Stünden, bevorzugt innerhalb von 10 bis 24 Stunden.

Palladiumkatalysatoren sind beispielsweise Bis(triphenylphosphin)palladium(II)chlorid, Tetrakis-(triphenylphosphin)palladium(0), Bis(tris-(o-tolyl)phosphino)palladium-(II)-chlorid oder ein aus Bis(acetonitril)dichlorpalladium oder Palladium(II)actat und einem Liganden, beispielsweise Tris-(o-tolyl)phosphin, Triphenylphosphin oder Diphenylphosphinoferrocen, hergestellter Palladiumkatalysator.

Basen sind beispielsweise 1,8-Diazabicyclo[5.4.0]undec-7-en (DBU), Triethylamin oder Diisopropylethylamin.

Inerte Lösungsmittel sind beispielsweise Ether wie 1,2-Dimethoxyethan, Dioxan, Glykoldimethylether oder Diethylenglykoldimethylether, Kohlenwasserstoffe wie Benzol, Xylol oder Toluol, oder andere Lösungsmittel wie Isobutyronitril, Acetonitril, Nitrobenzol, Dimethylformamid, Dimethylacetamid, Dimethylsulfoxid oder N-Methylpyrrolidon.

Bevorzugt ist ein aus Bis(acetonitril)dichlorpalladium und Tris-(o-tolyl)phosphin hergestellter Palladiumkatalysator und Triethylamin in Isobutyronitril.

Die Umsetzung einer Verbindung der Formel (VII) zu einer Verbindung der Formel (VIII) erfolgt durch Reaktion einer Verbindung der Formel (VII) mit einer Verbindung der Formel (IX) in Essigsäure in Gegenwart eines Palladiumkatalysators, eines Oxidationsmittels und einer Säure, in einem Temperaturbereich von 0°C bis 50°C, bevorzugt bei Raumtemperatur, bei Normaldruck, innerhalb von beispielsweise 5 bis 48 Stunden, bevorzugt innerhalb von 10 bis 24 Stunden.

Palladiumkatalysatoren sind beispielsweise Palladiumsalze wie Palladium(II)chlorid, Palladium(II)acetylacetonat, Palladium(II)acetat oder Natriumtetrachloropalladat, bevorzugt ist Palladium(II)acetat.

Oxidationsmittels sind beispielsweise p-Benzochinon oder Peroxide wie z.B. Wasserstoffperoxid, tert.-Butylhydroperoxid oder Natriumberporat, oder Schwefeltrioxid Pyridin Komplex, Mangandioxid, 2,3-Dichlor-5,6-dicyano-p-benzochinon (DDQ), Natriumperoxodisulfat oder Oleum (rauchende Schwefelsäure), bevorzugt ist p-Benzochinon, Natriumperoxodisulfat oder Oleum (rauchende Schwefelsäure) und besonders bevorzugt ist Oleum.

Säuren sind beispielsweise Methansulfonsäure, Trifluormethansulfonsäure oder substituierte Benzolsulfonsäuren, wie z. B. 4-Methylbenzolsulfonsäure, 4-Chlorbenzolsulfonsäure oder 4-Nitrobenzolsulfonsäure, oder konzentrierte Schwefelsäure in Form von Oleum, bevorzugt ist Trifluormethansulfonsäure oder Schwefelsäure in Form von Oleum, besonders bevorzugt ist Oleum.

Die Umsetzung einer Verbindung der Formel (VIII) zu einer Verbindung der Formel (V) erfolgt durch Reaktion einer Verbindung der Formel (VIII) mit einer Base in einem inerten Lösungsmittel, in einem Temperaturbereich von 40°C bis zum Rückfluss des Lösungsmittels, bevorzugt bei Rückfluss des Lösungsmittels, bei Normaldruck, innerhalb von beispielsweise 1 bis 48 Stunden, bevorzugt innerhalb von 2 bis 14 Stunden.

Basen sind beispielsweise Alkalicarbonate wie Cäsiumcarbonat, Natrium- oder Kaliumcarbonat, 1,8-Diazabicyclo[5.4.0]undec-7-en (DBU), Triethylamin oder Diisopropylethylamin, bevorzugt ist Kaliumcarbonat oder DBU.

Inerte Lösungsmittel sind beispielsweise Ether wie 1,2-Dimethoxyethan, Dioxan, Glykoldimethylether oder Diethylenglykoldimethylether, oder Kohlenwasserstoffe wie Benzol, Xylol oder Toluol, oder Ketone wie Aceton oder Methylisobutylketon (MIBK), oder andere Lösungsmittel wie Isobutyronitril, Acetonitril, Chlorbenzol, Nitrobenzol, Dimethylformamid, Dimethylacetamid, Dimethylsulfoxid, N-Methylpyrrolidon oder Tetrahydrothiophen-1,1-dioxid (Sulfolan), bevorzugt ist Aceton.

Des weiteren umfasst die vorliegende Erfindung ein Verfahren zur Enantiomerentrennung von {8-Fluor-2-[4-(3-methoxyphenyl)-piperazin-1-yl]-3-[2-methoxy-5-(trifluormethyl)phenyl]-3,4-dihydrochinazolin-4-yl}essigsäure-(C₁-C₄)-alkylester und Isolierung von (*S*)-{8-Fluor-2-[4-(3-methoxyphenyl)-piperazin-1-yl]-3-[2-methoxy-5-(trifluormethyl)phenyl]-3,4-di-hydrochinazolin-4-yl}essigsäure-(C₁-C₄)-alkylester, dadurch gekennzeichnet, dass der racemische Ester mit (2S,3S)-2,3-Bis[(4-methylbenzoyl)oxy]bemsteinsäure kristallisiert wird. Die Kristallisation erfolgt in einem Temperaturbereich von 0 bis 25°C in Ethylacetat. Das Salz des S-Enantiomers fällt zuerst aus der Lösung aus.

Bevorzugt ist ein Verfahren zur Enantiomerentrennung von {8-Fluor-2-[4-(3-Methoxyphenyl)-piperazin-1-yl]-3-[2-methoxy-5-(trifluormethyl)phenyl]-3,4-di-hydrochinazolin-4-yl} essigsäuremethylester und Isolierung von (S)-{8-Fluor-2-[4-(3-methoxyphenyl)-piperazin-1-yl]-3-[2-methoxy-5-(trifluormethyl)phenyl]-3,4-di-hydrochinazolin-4-yl}essigsäuremethylester, dadurch gekennzeichnet, dass der racemische Ester mit (2*S*,3*S*)-2,3-Bis[(4-methylbenzoyl)oxy]-bernsteinsäure kristallisiert wird. Die Kristallisation erfolgt in einem Temperaturbereich von 0 bis 25°C in Ethylacetat. Das Salz des S-Enantiomers fällt zuerst aus der Lösung aus.

Des weiteren umfasst die vorliegende Erfindung die (S)-{8-Fluor-2-[4-(3-methoxyphenyl)-piperazin-1-yl]-3-[2-methoxy-5-(trifluormethyl)-phenyl]-3,4-di-hydrochinazolin-4-yl}essigsäure-(C₁-C₄)-alkylester - (2*S*,3*S*)-2,3-Bis[(4-methylbenzoyl)oxy]bemsteinsäure-Salze.

Des weiteren umfasst die vorliegende Erfindung das (S)-{8-Fluor-2-[4-(3-methoxyphenyl)-piperazin-1-yl]-3-[2-methoxy-5-(trifluormethyl)-phenyl]-3,4-di-hydrochinazolin-4-yl}essigsäure-methylester - (2*S*,3*S*)-2,3-Bis[(4-methylbenzoyl)oxy]bernsteinsäure-Salz.

Des weiteren umfasst die vorliegende Erfindung ein Verfahren zur Racemisierung von (R)-{8-Fluor-2-[4-(3-methoxyphenyl)-piperazin-1-yl]-3-[2-methoxy-5-(trifluormethyl)phenyl]-3,4-dihydrochinazolin-4-yl}essigsäure-(C₁-C₄)-alkylester, dadurch gekennzeichnet, dass
in der ersten Stufe der Alkylester zur Säure verseift,
in der zweiten Stufe die Säure mit Natriummethylat oder Natriumethylat racemisiert und
in der dritten Stufe die Säure wieder zum Alkylester umgesetzt wird.

Bevorzugt ist ein Verfahren zur Racemisierung von (R)-{8-Fluor-2-[4-(3-methoxyphenyl)-piperazin-1-yl]-3-[2-methoxy-5-(trifluormethyl)phenyl]-3,4-di-hydrochinazolin-4-yl}essigsäure-methylester, dadurch gekennzeichnet, dass
in der ersten Stufe der Methylester zur Säure verseift,
in der zweiten Stufe die Säure mit Natriummethylat oder Natriumethylat racemisiert und
in der dritten Stufe die Säure wieder zum Methylester umgesetzt wird.

Die Verseifung in der ersten Stufe mit einer Säure oder einer Base erfolgt unter den gleichen Reaktionsbedingungen, wie die Verseifung des Esters einer Verbindung der Formel (II) zu einer Verbindung der Formel (I).

Die Racemisierung in der zweiten Stufe erfolgt durch Erhitzen der Säure mit Natriummethylat oder Natriumethylat, wobei bevorzugt mindestens 2 Äquivalente Base verwendet werden und wobei Natriummethylat oder Natriumethylat gegebenenfalls in einer alkoholischen Lösung eingesetzt werden, in einem Lösungsmittel unter Rückfluss, bei Normaldruck, für beispielsweise 36 bis 72 Stunden, bevorzugt für 50 bis 70 Stunden.

Lösungsmittel sind beispielsweise Ether wie 1,2-Dimethoxyethan, Dioxan, Glykoldimethylether oder Diethylenglykoldimethylether, Kohlenwasserstoffe wie Benzol, Xylol oder Toluol, oder andere Lösungsmittel wie Isobutyronitril, Acetonitril oder Dimethylsulfoxid, bevorzugt ist Acetonitril.

Die Veresterung in der dritten Stufe erfolgt beispielsweise durch Umsetzung der Säure mit Schwefelsäure in Methanol oder einem anderen Alkohol unter Rückfluss, bei Normaldruck, für beispielsweise 12 bis 48 Stunden, bevorzugt für 20 bis 30 Stunden.

Die im Rahmen des erfindungsgemäßen Verfahren beschriebenen Verbindungen können auch in Form ihrer Salze, Solvate oder Solvate der Salze vorliegen.

Die im Rahmen des erfindungsgemäßen Verfahren beschriebenen Verbindungen können in Abhängigkeit von ihrer Struktur in stereoisomeren Formen (Enantiomere, Diastereomere) existieren. Das erfindungsgemäße Verfahren umfasst deshalb auch die Herstellung und den Einsatz der Enantiomeren oder Diastereomeren und ihre jeweiligen Mischungen. Aus solchen Mischungen von Enantiomeren und/oder Diastereomeren lassen sich die stereoisomer einheitlichen Bestandteile in dem Fachmann bekannter Weise isolieren.

Die im Rahmen des erfindungsgemäßen Verfahren beschriebenen Verbindungen können in Abhängigkeit von der Struktur auch in Form ihrer Tautomere vorliegen. -

Als Salze sind im Rahmen der Erfindung physiologisch unbedenkliche Salze der im erfindungsgemäßen Verfahren eingesetzten und hergestellten Verbindungen bevorzugt.

Physiologisch unbedenkliche Salze der im erfindungsgemäßen Verfahren eingesetzten und hergestellten Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Äpfelsäure, Zitronensäure, Fumarsäure, Maleinsäure und Benzoesäure.

Physiologisch unbedenkliche Salze der im erfindungsgemäßen Verfahren eingesetzten und hergestellten Verbindungen umfassen auch Salze üblicher Basen, wie beispielhaft und vorzugsweise Alkalimetallsalze (z.B. Natrium- und Kaliumsalze), Erdalkalisalze (z.B. Calcium-und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Dihydroabiethylamin, Arginin, Lysin, Ethylendiamin und Methylpiperidin.

Als Solvate werden im Rahmen der Erfindung solche Formen der im erfindungsgemäßen Verfahren eingesetzten und hergestellten Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung bedeutet racemisch, dass die Verbindungen nicht enantiomerenrein vorliegen, d. h. die Verbindungen liegen als Gemische von (S)- und (R)-Enantiomer vor. Dabei ist das Verhältnis von (S)-Enantiomer zu (R)-Enantiomer variabel. Bevorzugt ist eine Mischung von (S)-Enantiomer zu (R)-Enantiomer von 1:1.

Im Rahmen der vorliegenden Erfindung haben die Substituenten, soweit nicht anders spezifiziert, die folgende Bedeutung:

Alkyl per se und "Alk" und "Alkyl" in Alkoxy, Alkylamino, Alkylthio, Alkylcarbonyl, Alkylsulfonyl, Alkoxycarbonyl und Alkylaminosulfonyl stehen für einen linearen oder verzweigten Alkylrest mit in der Regel 1 bis 6 ("C₁-C₆-Alkyl"), vorzugsweise 1 bis 4, besonders bevorzugt 1 bis 3 Kohlenstoffatomen, beispielhaft und vorzugsweise für Methyl, Ethyl, n-Propyl, Isopropyl, tert.-Butyl, n-Pentyl und n-Hexyl.

Alkoxy steht beispielhaft und vorzugsweise für Methoxy, Ethoxy, n-Propoxy, Isopropoxy, tert.-Butoxy, n-Pentoxy und n-Hexoxy.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, tert.-Butylamino, n-Pentylamino, n-Hexylamino, *N,N*-Dimethylamino, *N,N-*Diethylamino, *N*-Ethyl-*N*-methylamino, *N*-Methyl-N-n-propylamino, *N*-Isopropyl-*N*-n-propylamino, *N*-t-Butyl-*N*-methylamino, *N*-Ethyl-*N*-n-pentylamino und *N*-n-Hexyl-*N*-methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Alkylthio steht beispielhaft und vorzugsweise für Methylthio, Ethylthio, n-Propylthio, Isopropylthio, tert.-Butylthio, n-Pentylthio und n-Hexylthio.

Alkylcarbonyl steht beispielhaft und vorzugsweise für Methylcarbonyl, Ethylcarbonyl, n-Propylcarbonyl, Isopropylcarbonyl, tert.-Butylcarbonyl, n-Pentylcarbonyl und n-Hexylcarbonyl.

Alkylsulfonyl steht beispielhaft und vorzugsweise für Methylsulfonyl, Ethylsulfonyl, n-Propylsulfonyl, Isopropylsulfonyl, tert.-Butylsulfonyl, n-Pentylsulfonyl und n-Hexylsulfonyl.

Alkoxycarbonyl steht beispielhaft und vorzugsweise für Methoxycarbonyl, Ethoxycarbonyl, n-Propoxycarbonyl, Isopropoxycarbonyl, tert.-Butoxycarbonyl, n-Pentoxycarbonyl und n-Hexoxycarbonyl.

Alkylaminosulfonyl steht für einen Alkylaminosulfonylrest mit einem oder zwei (unabhängig voneinander gewählten) Alkylsubstituenten, beispielhaft und vorzugsweise für Methylaminosulfonyl, Ethylaminosulfonyl, n-Propylaminosulfonyl, Isopropylaminosulfonyl, tert.-Butylaminosulfonyl, n-Pentylaminosulfonyl, n-Hexylaminosulfonyl, *N,N*-Dimethylaminosulfonyl, *N,N*-Diethylaminosulfonyl, *N*-Ethyl-*N*-methylaminosulfonyl, *N*-Methyl-N-n-propylaminosulfonyl, *N*-Isopropyl-*N* n-propylaminosulfonyl, *N*-tert.-Butyl-*N*-methylaminosulfonyl, *N*-Ethyl-N-n-pentylamino-sulfonyl und *N*-n-Hexyl-*N*-methylaminosulfonyl. C₁-C₃-Alkylaminosulfonyl steht beispielsweise für einen Monoalkylaminosulfonylrest mit 1 bis 3 Kohlenstoffatomen oder für einen Dialkylaminosulfonylrest mit jeweils 1 bis 3 Kohlenstoffatomen pro Alkylsubstituent.

Aryl steht für einen mono- oder bicyclischen aromatischen, carbocyclischen Rest mit in der Regel 6 bis 10 Kohlenstoffatomen; beispielhaft und vorzugsweise für Phenyl und Naphthyl.

Halogen steht für Fluor, Chlor, Brom und Jod.

Die vorliegende Erfindung wird nachstehend anhand von nicht einschränkenden bevorzugten Beispielen und Vergleichsbeispielen näher dargestellt. Soweit nicht anderweitig angegeben, beziehen sich alle Mengenangaben auf Gewichtsprozente.

### Ausführungsbeispiele

### Abkürzungsverzeichnis:

- ACN: Acetonitril
- API-ES-pos.: Atmospheric pressure ionization, electrospray, positive (bei MS)
- API-ES-neg.: Atmospheric pressure ionization, electrospray, negative (bei MS)
- ca.: circa
- CI, NH₃: chemische Ionisierung (mit Ammoniak)
- DBU: 1,8-Diazabicycto[5.4.0]undec-7-en
- DMAP: 4-(Dimethylamino)pyridin
- DMSO: Dimethylsulfoxid
- ESTD: externe Standardisierung
- h: Stunde(n)
- HPLC: Hochdruckflüssigchromatographie (high pressure liquid chromatography)
- konz.: konzentriert(e)
- MIBK: Methylisobutylketon
- min.: Minuten
- MS: Massenspektroskopie
- NMR: Kernresonanzspektroskopie (nuclear magnetic resonance spectroscopy)
- R_{T}: Retentionszeit (bei HPLC)
- VTS: Vakuumtrockenschrank

### Allgemeine Methoden HPLC:

Methode 1 (HPLC): Instrument: HP 1050 mit Multiple Wavelength-Detektion; Säule: Phenomenex-Prodigy ODS (3) 100A, 150 mm x 3 mm, 3 µm; Eluent A: (1,0 g KH₂PO₄ + 1,0 mL H₃PO₄) / L Wasser, Eluent B: Acetonitril; Gradient: 0 min 10% B, 25 min 80% B, 35 min 80% B; Fluss: 0,5 mL/min; Temp.: 45 °C; UV-Detektion: 210 nm.

Methode 2 (HPLC): Instrument: HP 1050 mit variabler Wellenlängen-Detektion; Säule: Chiral AD-H, 250 mm x 4,6 mm, 5 µm; Eluent A: n-Heptan + 0,2 % Diethylamin, Eluent B: Isopropanol + 0,2 % Diethylamin; Gradient: 0 min 12,5 % B, 30 min 12,5 % B; Fluss: 1 mL/min; Temp.: 25°C; UV-Detektion: 250 nm.

Für das S-Enantiomer werden positive, für das R-Enantiomer negative e.e.-Werte angegeben.

**Methode 3 (HPLC)**: Instrument: HP 1050 mit variabler Wellenlängen-Detektion; Säule: Chiral AD-H, 250 mm x 4,6 mm, 5 µm; Eluent A: *n*-Heptan + 0,2 % Diethylamin, Eluent B: Isopropanol + 0,2 % Diethylamin; Gradient: 0 min 25 % B, 15 min 25 % B; Fluss: 1 mL/min; Temp.: 30 °C**;** UV-Detektion: 250 nm.

Für das-S-Enantiomer werden positive, für das R-Enantiomer negative e.e.-Werte angegeben.

**Methode 4 (HPLC)**: Instrument: HP 1100 mit variabler Wellenlängen-Detektion; Säule: Phenomenex-Prodigy C8, 150 mm x 3 mm, 5 µm; Eluent A: (1,36 g KH₂PO₄ + 1,15 g 85 %ige H₃PO₄) / L Wasser, Eluent B: Acetonitril; Gradient: 0 min 10% B, 20 min 80% B, 30 min 80% B; Fluss: 0,5 mL/min; Temp.: 40 °C; UV-Detektion: 210 nm.

Angegebene Ausbeuten sind nicht gehaltskorrigiert.

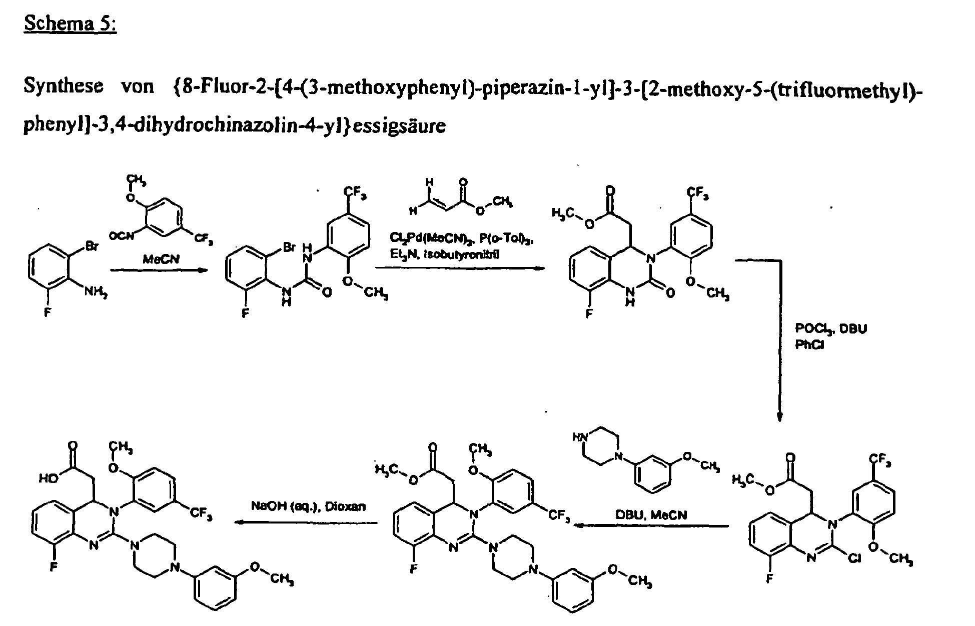

### Beispiel 1

### N-(2-Brom-6-fluorphenyl)-N'-[2-methoxy-5-(trifluormethyl)phenyl]harnstoff

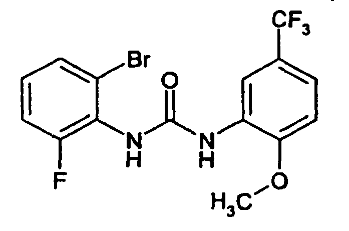

2-Methoxy-5-trifluormethylphenylisocyanat (274,3 g) werden in Acetonitril (1 L) gelöst, dann wird 2-Brom-6-fluoranilin (200 g) dazugegeben und mit Acetonitril (50 mL) nachgespült. Die resultierende klare Lösung wird 38 h bei Rückfluss (ca. 85 °C) gerührt, dann im Vakuum bei 40 °C zu einem zähen Brei aufkonzentriert. Dieser wird abgesaugt, mit Acetonitril (260 mL, auf 0-5 °C gekühlt) nachgewaschen und über Nacht bei 45 °C im VTS mit Schleppstickstoff getrocknet. So werden insgesamt 424,3 g -N*-(2-Brom-6-fluorphenyl)*-N'-*[2-methoxy-5-(trifluormethyl)phenyl] harnstoff* als Feststofferhalten, entsprechend 99,2 % der Theorie.

¹H NMR (300 MHz, d₆-DMSO): δ = 8,93 (s, 1H), 8,84 (s, 1H), 8,52 (d, ³*J*= 2,3, 2H), 7,55 (d, ²*J* 7,7, 1H), 7,38-7,26 (m, 3H), 7,22 (d, ²*J*= 8,5, 1H), 4,00 (s, 3H) ppm;
MS (API-ES-pos.): m/z = 409 [(M+H)⁺, 100 %];
HPLC (Methode 1): R_{T} = 22,4 und 30,6 min.

### Beispiel 2

### N-(2-Brom-6-fluorphenyl)-N'-[2-methoxy-5-(trifluormethyl)phenyl]harnstoff (Alternativsynthese)

2-Methoxy-5-trifluormethylphenylisocyanat (1,19 kg) werden bei ca. 35 °C aufgeschmolzen und in Acetonitril (4,2 L) gelöst, dann wird 2-Brom-6-fluoranilin (870 g) dazugegeben und mit Acetonitril (380 mL) nachgespült. Die resultierende klare Lösung wird 45 h bei 74-88 °C gerührt, dann im Vakuum (200 mbar) bei 50 °C zu einem zähen Brei aufkonzentriert (Destillatmenge 4,4 L). Dieser wird bei Raumtemperatur mit Diisopropylether (1,5 L) verdünnt, abgesaugt, mit Diisopropylether (1,15 L) nachgewaschen und bei 45 °C im VTS mit Schleppstickstoff bis zur Massenkonstanz (24 h) getrocknet. So werden insgesamt 1,63 kg N-*(2-Brom-6-fluorphenyl)*-N'-*[2-methoxy-5-(trifluormethyl)phenyl]harnstoff* als Feststoff erhalten, entsprechend 87,5 % der Theorie.
HPLC (Methode 1): R_{T} = 22,6 und 30,8 min.

### Beispiel 3

### {8-Fluor-3-[2-methoxy-5-(trifluormethyl)phenyl]-2-oxo-1,2,3,4-tetrahydrochinazolin-4-yl}essigsäuremethylester

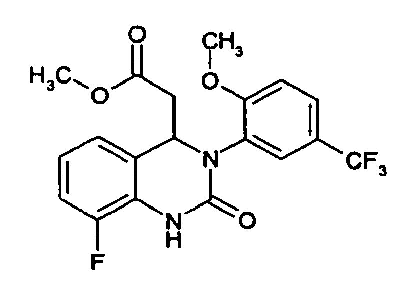

*N*-(2-Brom-6-fluorphenyl)-*N*'-[2-methoxy-5-(trifluormethyl)phenyl]harnstoff (300 g) wird unter einer Stickstoff-Atmosphäre in Isobutyronitril (1,2 L) suspendiert, dann werden Triethylamin (210 mL), Bis(acetonitril)dichlorpalladium (7,5 g), Tris-(o-tolyl)phosphin (18,0 g) und Methylacrylat (210 mL) in dieser Reihenfolge zugegeben. Die resultierende Suspension wird 16 h bei Rückfluss (ca. 102 °C) gerührt und dann auf Raumtemperatur gekühlt. Wasser (1,2 L) wird zugegeben und das Gemisch 1 h bei Raumtemperatur gerührt, dann abgesaugt und mit Wasser/Methanol (1:1, 300 mL) und Acetonitril (100 mL) gewaschen. Der Rückstand wird über Nacht bei 45 °C im VTS mit Schleppstickstoff getrocknet. So werden insgesamt 208 g *{8-Fluor-3-[2-methoxy-5-(trif*/*uormethy*/*)pheny*/*]-2-oxo-1,2,3,4-tetrahydrochinazolin-4-yl}essigsäure-methylester* als Feststoff erhalten, entsprechend 68,5 % der Theorie.

¹H NMR (300 MHz, d₆-DMSO): δ = 9,73 (s, 1H), 7,72 (d, ²*J* = 7,3, 1H), 7,71 (s, 1H), 7,33 (d, ²*J*= 9,3, 1H), 7,15 (dd, ²*J* = 9,6, ²*J* = 8,6, 1H), 7,01 (d, ²*J*= 7,3, 1H), 6,99-6,94 (m, 1H), 5,16 (t, ²*J*= 5,9, 1H), 3,84 (s, 3H), 3,41 (s, 3H), 2,81 (dd, ²*J* = 15,4, ²*J*= 5,8, 1H) , 2,62 (dd, ²*J*= 15,4, ²*J*= 6,3, 1H) ppm;
MS (API-ES-pos.): m/z = 413 [(M+H)⁺, 100 %], 825 [(2M+H)⁺, 14 %];
HPLC (Methode 1): R_{T} = 19,3 min; Pd (ICP): 16.000 ppm.

### Beispiel 4

### {8-Fluor-3-[2-methoxy-5-(trifluormethyl)phenyl]-2-oxo-1,2,3,4-tetrahydrochinazolin-4-yl}essigsäuremethylester (Alternativsynthese)

*N*-(2-Brom-6-fluorphenyl)-*N*'-[2-methoxy-5-(trifluormethyl)phenyl]harnstoff (2,5 kg) wird unter einer Stickstoff-Atmosphäre in Isobutyronitril (9 L) suspendiert, dann werden Triethylamin (1,31 kg), Bis(acetonitril)dichlorpalladium (64,9 g), Tris-(o-tolyl)phosphin (149 g) und Methylacrylat (1,59 kg) in dieser Reihenfolge zugegeben. Die resultierende Suspension wird 22 h bei 90-100 °C gerührt, dann auf Raumtemperatur gekühlt. Wasser (9 L) wird zugegeben und das Gemisch 1 h bei Raumtemperatur gerührt, dann abgesaugt und mit Wasser/Methanol (1:1, 2,5 L) und Acetonitril (850 mL) gewaschen. Der Rückstand wird über Nacht bei 45 °C im VTS mit Schleppstickstoff bis zur Massenkonstanz (21 h) getrocknet. So werden insgesamt 1,90 kg *{*8-*Fluor-3-[2-methoxy-5-(trif*/*uormethy*/*)pheny*/*]-2-oxo-1,2,3,4-tetrahydrochinazo*/*in-4-y*/*}essigsäuremethylester* als Feststoff erhalten, entsprechend 74,9 % der Theorie.
HPLC (Methode 1): R_{T} = 19,4 min.

### Beispiel 5

### {2-Chlor-8-fluor-3-[2-methoxy-5-(trifluormethyl)phenyl]-3,4-dihydrochinazolin-4-yl}essigsäure-methylester / Chlorierung

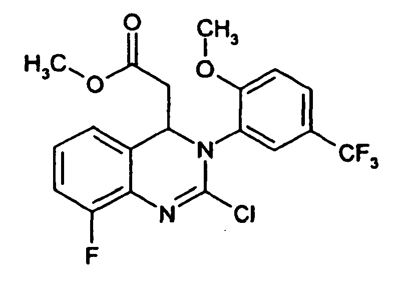

Eine Lösung von 2,84 kg {8-Fluor-3-[2-methoxy-5-(trifluormethyl)phenyl]-2-oxo-1,2,3,4-tetrahydrochinazolin-4-yl}essigsäuremethylester in 14,8 L Chlorbenzol wird auf Rückfluss erhitzt und das Lösungsmittel wird solange abdestilliert, bis sich kein Wasser mehr abscheidet. Es wird auf 120°C abgekühlt. Innerhalb 10 min dosiert man 3,17 kg Phosphoroxychlorid, und anschließend wird innerhalb weiterer 10 min 2,10 kg DBU zugegeben. Es wird 9 Stunden auf Rückfluss erhitzt.

Zur Aufarbeitung kühl man auf 40°C ab, rührt über Nacht und dosiert den Kesselinhalt auf 11,4 L Wasser, das zuvor auf 40°C temperiert wurde. Bei der Dosierung soll eine Innentemperatur von 40-45°C eingehalten werden. Man lässt auf Raumtemperatur abkühlen, gibt 11,4 L Dichlormethan zu, filtriert über eine Seitz Filterplatte und trennt die Phasen. Die organische Phase wird mit 11,4 L Wasser, 11,4 L einer wässrigen gesättigten Natriumhydrogencarbonat-Lösung und erneut mit 11,4 L Wasser gewaschen. Die organische Phase wird am Rotationsverdampfer im Vakuum aufkonzentriert und der verbleibende Rückstand (2,90 kg) wird ohne weitere Behandlung in der nächsten Stufe eingesetzt.

¹H NMR (300 MHz, d₆-DMSO): δ = 7,93-7,82 (m, 2H), 7,38 (d, ²*J*= 8,9, 1H), 7,17 (m, 2H), 6,97-6,91 (m, 1H), 5,45 und 5,29 (m und t, ²*J* = 5,4, 1H), 3,91 und 3,84 (2s, 3H), 3,48 (s, 3H), 3,0-2,6 (m, 2H) ppm;
MS (CI, NH₃): m/z = 431 [(M+H)⁺, 100 %];
HPLC (Methode 1): R_{T} = 23,5 min; typischer Pd-Wert (ICP): 170 ppm.

### Beispiel 6

### {8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester / Aminierung

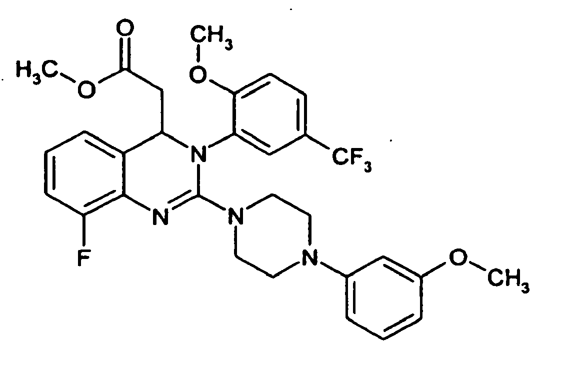

{2-Chlor-8-fluor-3-[2-methoxy-5-(trifluormethyl)phenyl]-3,4-dihydrochinazolin-4-yl}essigsäure-methylester (52,5 g) wird in 1,4-Dioxan (100 mL) gelöst, dann werden bei Raumtemperatur 3-Methoxyphenylpiperazin (25,8 g) und DBU (20,4 g) zugegeben, wobei die Temperatur ansteigt. Das Gemisch wird 22 h bei Rückfluss gerührt, dann auf Raumtemperatur gekühlt, mit Ethylacetat (500 mL) und Wasser (200 mL) verdünnt und die Phasen getrennt. Die organische Phase wird mit 0,2N Salzsäure (dreimal 100 mL) und Wasser (200 mL) gewaschen, über Natriumsulfat getrocknet und einrotiert. So werden insgesamt 62,5 g *{8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester* als erstarrter Schaum erhalten, der als Rohprodukt ohne weitere Reinigung umgesetzt wird.
HPLC (Methode 1): R_{T} = 16,6 min.

### Beispiel 7

{8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester / Eintopf Chlorierung + Aminierung

{8-Fluor-3-[2-methoxy-5-(trifluormethyl)phenyl]-2-oxo-1,2,3,4-tetrahydrochinazolin-4-yl}essig-säuremethylester (50,0 g) wird in Chlorbenzol (300 mL) vorgelegt, dann wird Chlorbenzol teilweise abdestilliert (50 mL). Das Gemisch wird auf 120 °C gekühlt, DBU (36,9 g) wird zugegeben, dann wird bei 120-128 °C Phosphoroxychlorid (33,4 mL) über 10 min. zudosiert. Das Gemisch wird 9 h bei Rückfluss (ca. 130 °C) gerührt. Anschließend wird auf 40 °C gekühlt, langsam bei 40-45 °C mit Wasser (200 mL) versetzt, auf Raumtemperatur gekühlt und mit Dichlormethan (200 mL) verdünnt, ausgerührt und dann die Phasen getrennt. Die organische Phase wird mit Wasser (200 mL), gesättigter wässriger Natriumhydrogencarbonat-Lösung (200 mL) und nochmals Wasser (200 mL) gewaschen, über Natriumsulfat getrocknet, einrotiert und dann im Hochvakuum bei 50 °C getrocknet. Der Rückstand (48,1 g) wird in Chlorbenzol (20 mL) gelöst, dann wird mit 1,4-Dioxan (80 mL) verdünnt und bei Raumtemperatur 3-Methoxyphenylpiperazin (23,6 g) und DBU (18,7 g) zugegeben, wobei die Temperatur ansteigt. Das Gemisch wird 22 h bei Rückfluss gerührt, dann auf Raumtemperatur gekühlt, mit Ethylacetat (500 mL) und Wasser (200 mL) verdünnt und die Phasen getrennt. Die organische Phase wird mit 0,2N Salzsäure (dreimal 100 mL) und Wasser (200 mL) gewaschen, über Natriumsulfat getrocknet und einrotiert. So werden insgesamt 55,6 g *{8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester* als erstarrter Schaum erhalten, der als Rohprodukt ohne weitere Reinigung umgesetzt wird.
HPLC (Methode 1): R_{T} = 16,2 min.

### Beispiel 8

### (±)-{8-Fluor-2-[4-(3-methoxyphenyl)piperazin-1-yl]-3-(2-methoxy-5-trifluonmethylphenyl)-3,4-dihydrochinazolin-4-yl}essigsäure / Verseifung Racemat

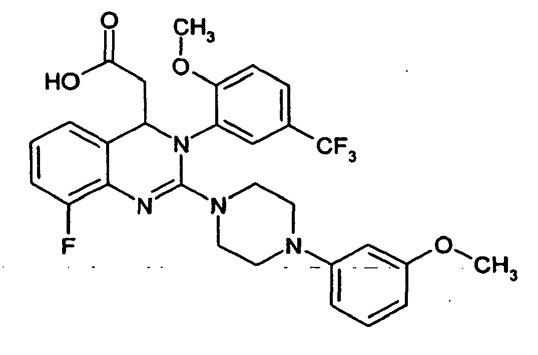

{8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester (64 g) wird in 1,4-Dioxan (450 mL) und 1N Natronlauge (325 mL) gelöst und 2 h bei Raumtemperatur gerührt, dann wird im Vakuum bei 30 °C ein Teil des Lösungsmittels abdestilliert (400 mL). Anschließend wird Toluol (300 mL) zugegeben und die Phasen getrennt. Die wässrige Phase wird mit Toluol (zweimal 150 mL) gewaschen, dann werden die vereinten organischen Phasen nochmals mit 1N Natronlauge (50 mL) extrahiert. Der pH-Wert der vereinten wässrigen Phasen wird mit 2N Salzsäure (ca. 150 mL) auf 7,5 gestellt, dann wird MIBK (150 mL) zugegeben. Die Phasen werden getrennt, die wässrige Phase nochmals mit MIBK (150 mL) extrahiert, dann die vereinten MIBK-Phasen über Natriumsulfat getrocknet und bei 45 °C eingeengt. So werden insgesamt 64 g *(±)-{8-Fluor-2-[4-(3-methoxyphenyl)piperazin-1-y*/*]-3-(2-methoxy-5-trif*/*uormethylpheny*/*)-*3,*4-dihydrochinazolin-4-yl}essigsäure* als amorpher Feststoff in quantitativer Ausbeute erhalten.
HPLC (Methode 1): R_{T} = 14,9 min.

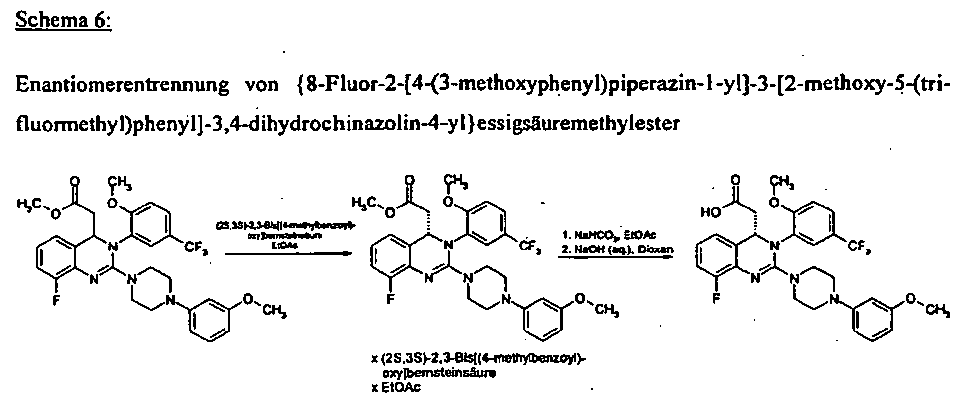

### Beispiel 9

### (2S,3S)-2,3-Bis[(4-methylbenzoyl)oxy]bernsteinsäure -{8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester (1:1-Salz) / Kristallisation

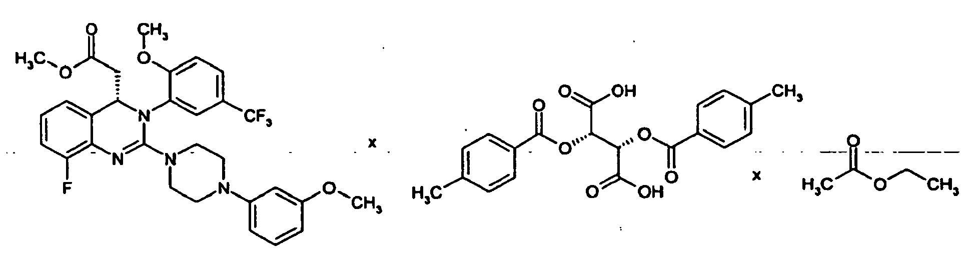

{8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester (62,5 g, Rohprodukt) wird in Ethylacetat (495 mL) gelöst und filtriert. Zum Filtrat wird (2*S*,3*S*)-2,3-Bis[(4-methylbenzoyl)oxy]bernsteinsäure (42,0 g) gegeben, das Gemisch 30 min. bei Raumtemperatur gerührt, dann wird mit (2*S*,3*S*)-2,3-Bis[(4-methylbenzoyl)oxy]-bernsteinsäure - {8-Fluor-2-[4-(3-methoxyphenyl)piperazin-1-yl]-3-[2-methoxy-5-(trifluormethyl)-phenylJ-3,4-dihydrochinazolin-4-yl}essigsäuremethylester (1:1-Salz) (165 mg) angeimpft und 3 Tage bei Raumtemperatur gerührt, anschließend auf 0-3 °C gekühlt und weitere 3 h gerührt. Die Suspension wird abgesaugt und mit kaltem Ethylacetat (0-10 °C, 35 mL) nachgewaschen. Die Kristalle werden 18 h bei 40 °C im VTS mit Schleppstickstoff getrocknet. So werden insgesamt 37,1 g des Salzes als Feststoff erhalten, entsprechend 30,4 % der Theorie über drei Stufen (Chlorierung, Aminierung und Kristallisation) bezogen auf das Racemat, oder 60,8 % bezogen auf das entstandene S-Enantiomer.

¹H NMR (300 MHz, d₆-DMSO): δ = 7,90 (d, ²*J* = 7,8, 4H), 7,56 (d, ²*J* = 8,3, 1H), 7,40 (d, ²*J* = 7,8, 4H), 7,28-7,05 (m, 4H), 6,91-6,86 (m, 2H), 6,45 (d, ²*J*=8,3, 1H), 6,39-6,36 (m, 2H), 5,82 (s, 2H), 4,94 (m, 1H), 4,03 (q, ²*J* = 7,1, 2H), 3,83 (brs, 3H), 3,69 (s, 3H), 3,64 (s, 3H), 3,47-3,36 (m, 8H und Wasser, 2H), 2,98-2,81 (m, 5H), 2,58-2,52 (m, 1H), 2,41 (s, 6H), 1,99 (s, 3H), 1,18 (t, ²*J*=7,2, 3H) ppm;
HPLC (Methode 1): R_{T} = 16,6 und 18,5 min.

### Beispiel 10

### (2S,3S)-2,3-Bis[(4-methylbenzoyl)oxy]bernsteinsäure -{8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester (1:1-Salz) / Umkristallisation

(2S,3S)-2,3-Bis[(4-methylbenzoyl)oxy]bernsteinsäure - {8-Fluor-2-[4-(3-methoxyphenyl)piperazin-1-yl]-3-[2-methoxy-5-(trifluormethyl)phenyl]-3,4-dihydrochinazolin-4-yl}essigsäuremethyl-ester (1:1-Salz) (36,8 g) wird in Ethylacetat (370 mL) suspendiert und durch. Erhitzen auf Rückfluss (77 °C) gelöst. Das Gemisch wird langsam auf Raumtemperatur gekühlt. Dabei erfolgt eine spontane Kristallisation. Die Suspension wird 16 h bei RT gerührt, anschließend auf 0-5 °C gekühlt und weitere 3 h gerührt. Die Suspension wird abgesaugt und mit kaltem Ethylacetat (0-10 °C, zweimal 15 mL) nachgewaschen. Die Kristalle werden 18 h bei 45 °C im VTS mit Schleppstickstoff getrocknet. So werden insgesamt 33,6 g des Salzes als Feststoff erhalten, entsprechend 91,3% der Theorie.
HPLC (Methode 1): R_{T} = 16,9 und 18,8 min.;
HPLC (Methode 3): 99,9 % e.e.

### Beispiel 11

### (S)-{8-Fluor-2-[4-(3-methoxyphenyl)piperazin-1-yl]-3-(2-methoxy-5-trifluormethylphenyl)-3,4-dihydrochinazolin-4-yl}essigsäure

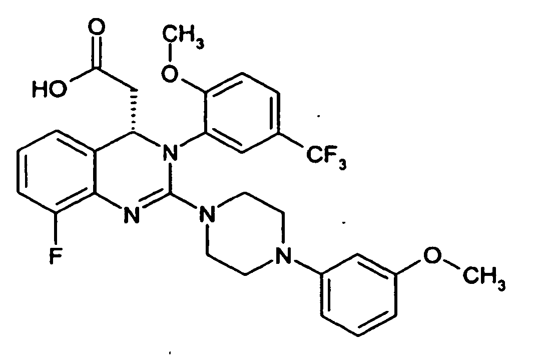

(2*S*,3*S*)-2,3-Bis[(4-methylbenzoyl)oxy]bernsteinsäure -{8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester (1:1-Salz) (10,1 g, enthaltend 14 ppm Pd) werden in Ethylacetat (100 mL) suspendiert und solange mit gesättigter wässriger Natriumbicarbonatlösung (100 mL) geschüttelt, bis beide Phasen klar werden. Die Phasen werden getrennt, die organische Phase wird einrotiert. Der Rückstand wird in 1,4-Dioxan (100 mL) und 1N Natronlauge (31,2 mL) gelöst und 3 h bei Raumtemperatur gerührt. Anschließend wird der pH-Wert mit 1N Salzsäure (ca. 17 mL) auf 7,5 gestellt, MIBK (80 mL) zugegeben, dann der pH-Wert mit 1N Salzsäure (ca. 2 mL) auf 7,0 nachgestellt. Die Phasen werden getrennt, die organische Phase über Natriumsulfat getrocknet und eingeengt. Der Rückstand wird in Ethanol (40 mL) gelöst und eingeengt, dann nochmals in Ethanol (40 mL) gelöst und eingeengt und im Hochvakuum bei 50 °C getrocknet. Der erstarrte Schaum wird 18 h bei 45 °C im VTS mit Schleppstickstoff getrocknet. So werden insgesamt 5,05 g *(S)-{8-Fluor-2-[4-(3-methoxyphenyl)piperazin-1-yl]-3-(2-methoxy-5-trifluormethylphenyl}-3,4-dihydrochinazolin-4-yl}essigsäure* als amorpher Feststoff erhalten, entsprechend 85,0 % der Theorie.

¹H NMR (300 MHz, d₆-DMSO): δ = 7,53 (d, ²*J* = 8,4, 1H), 7,41 (brs, 1H), 7,22 (d, ²*J* = 8,5, 1H), 7,09-7,01 (m, 2H), 6,86 (m, 2H), 6,45 (dd, ²*J* = 8,2, ³*J* = 1,8, 1H), 6,39-6,34 (m, 2H), 4,87 (t, ²*J* = 7,3, 1H), 3,79 (brs, 3H), 3,68 (s, 3H), 3,50-3,38 (m, 4H), 2,96-2,75 (m, 5H), 2,45-2,40 (m, 1H) ppm;
MS (API-ES-neg.): m/z = 571 [(M-H), 100 %];
HPLC (Methode 1): R_{T} = 15,1 min;
HPLC (Methode 2): 99,8 % e.e.; Pd (ICP): <1 ppm.

### Beispiel 12

### (2R,3R)-2,3-Bis[(4-methylbenzoyl)oxy]bernsteinsäure-{8-Fluor-2-[4-(3-methoxyphenyl)piperazin-1-yl]-3-[2-methoxy-5-(trifluormethyl)phenyl]-3,4-dihydrochinazolin-4-yl}essigsäuremethyl-ester (1:1-salz) / Kristallisation R-Isomer aus Mutterlauge

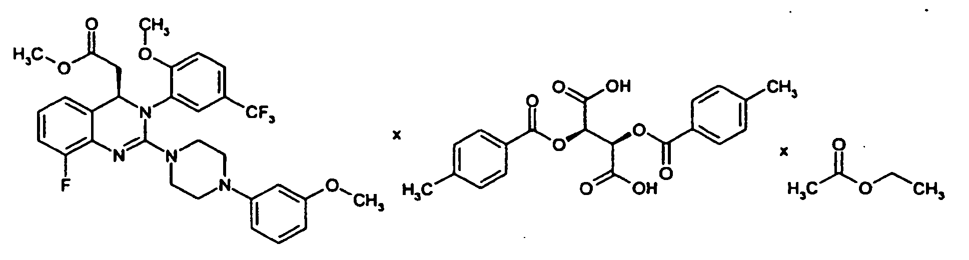

Die Mutterlauge aus einer Kristallisation von (2*S*,3*S*)-2,3-Bis[(4-methylbenzoyl)oxy]-bernsteinsäure - {8-Fluor-2-[4-(3-methoxyphenyl)piperazin-1-yl]-3-[2-methoxy-5-(trifluormethyl)-phenyl]-3,4-dihydrochinazolin-4-yl}essigsäuremethylester (1:1-Salz) im 279 g-Maßstab wird mit gesättigter wässriger Natriumbicarbonatlösung (1,5 L) geschüttelt, die Phasen werden getrennt und die organische Phase wird mit halbgesättigter wässriger Natriumbicarbonatlösung (1,5 L) geschüttelt. Die Phasen werden getrennt, die organische Phase über Natriumsulfat getrocknet und einrotiert. Der Rückstand (188,4 g) wird in Ethylacetat (1,57 L) gelöst, dann wird (2*R*,3*R*)-2,3-Bis[(4-methylbenzoyl)oxy]bernsteinsäure (121,7 g) zugegeben und das Gemisch 10 min. bei Raumtemperatur gerührt. Anschließend wird mit (2*R*,3*R*)-2,3-Bis[(4-methyl-benzoyl)oxy]bernsteinsäure-{8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester (1:1-Salz) (0,38 g) angeimpft und 18 h bei Raumtemperatur gerührt, anschließend auf 0-3 °C gekühlt und weitere 3 h gerührt. Die Suspension wird abgesaugt und mit kaltem Ethylacetat (0-10 °C, 500 mL) nachgewaschen. Die Kristalle werden 18 h bei 40 °C im VTS mit Schleppstickstoff getrocknet. So werden insgesamt 160 g des Salzes als Feststoff erhalten.
HPLC (Methode 1): R_{T}= 16,6 und 18,5 min.;
HPLC (Methode 3): -99,0 % e.e.

### Beispiel 13

### (R)-{8-Fluor-2-[4-(3-methoxyphenyl)piperazin-1-yl]-3-(2-methoxy-5-trifluormethylphenyl)-3,4-dihydrochinazolin-4-yl}essigsäure / Herstellung R-Isomer

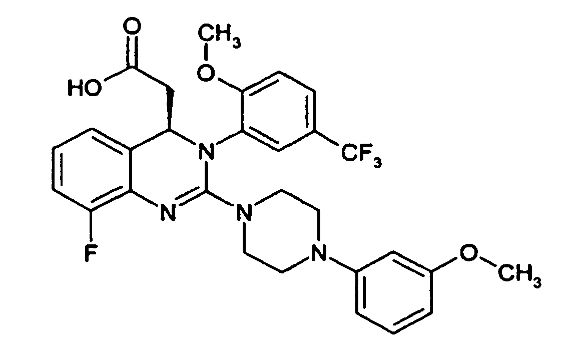

(2*R*,3*R*)-2,3-Bis[(4-methylbenzoyl)oxy]bernsteinsäure - {8-Fluor-2-[4-(3-methoxyphenyl)piperazin-1-yl]-3-[2-methoxy-5-(trifluormethyl)phenyl]-3,4-dihydrochinazolin-4-yl}essigsäuremethyl-ester (1:1-Salz) (170 g) werden in Ethylacetat (850 mL) suspendiert und solange mit gesättigter wässriger Natriumbicarbonatlösung (850 mL) geschüttelt, bis beide Phasen klar werden (ca. 5 min.). Die Phasen werden getrennt, das Lösungsmittel der organischen Phase wird bei Normaldruck mit 1,4-Dioxan bis zu einer Endtemperatur von 99 °C getauscht (portionsweise insgesamt 2,55 L Lösungsmittel abdestilliert und 2,55 L 1,4-Dioxan eingesetzt). Das Gemisch wird auf Raumtemperatur gekühlt und 18 h bei Raumtemperatur mit 1N Natronlauge (525 mL) gerührt. Anschließend wird der pH-Wert mit konzentrierter Salzsäure (ca. 35 mL) auf 7,5 gestellt, MIBK (850 mL) zugegeben, dann der pH-Wert mit konzentrierter Salzsäure (ca. 10 mL) auf 7,0 nachgestellt. Die Phasen werden getrennt, die organische Phase über Natriumsulfat getrocknet und eingeengt. Der Rückstand wird in Ethanol (350 mL) gelöst und eingeengt, dann nochmals in Ethanol (350 mL) gelöst und bei 50 °C eingeengt. So werden insgesamt 91,6 g *(R)-{8-Fluor-2-[4-(3-methoxyphenyl)piperazin-1-yl]-3-(2-methoxy-5-trifluormethylphenyl)-3,4-dihydrochinazolin-4-yl}essigsäure* als amorpher Feststoff erhalten, entsprechend 91,6 % der Theorie.
HPLC (Methode 1): R_{T} = 14,8 min.

### Beispiel 14

### {8-Fluor-2-[4-(3-methoxyphenyl)piperazin-1-yl]-3-(2-methoxy-5-trifluormethylphenyl)-3,4-dihydrochinazolin-4-yl}essigsäure / Racemisierung R-Enantiomer

(*R*)-{8-Fluor-2-[4-(3-methoxyphenyl)piperazin-1-yl]-3-(2-methoxy-5-trifluormethylphenyl)-3,4-dihydrochinazolin-4-yl}essigsäure (50 g) wird in Acetonitril (500 mL) gelöst und mit Natriummethylat (30 %ig in Methanol, 32,4 mL) versetzt und dann 60 h bei Rückfluss gerührt. Nach Abkühlen auf Raumtemperatur wird das Gemisch im Vakuum auf die Hälfte aufkonzentriert, dann wird mit Salzsäure (20 %ig, ca. 20 mL) auf pH 7,5 gestellt, MIBK (200 mL) zugegeben und mit Salzsäure (20 %ig) auf pH 7 nachgestellt. Die Phasen werden getrennt, die organische Phase über Natriumsulfat getrocknet und zum harten Schaum einrotiert. Der Rückstand wird in Ethanol (150 mL) gelöst und eingeengt, dann nochmals in Ethanol (150 mL) gelöst und eingeengt. So werden insgesamt 54,2 g *(±)-{8-Fluor-2-[4-(3-methoxyphenyl)piperazin-1-yl]-3-(2-methoxy-5-trifluormethylphenyl)-3,4-dihydrochinazolin-4-yl}essigsäure* als amorpher Feststoff in quantitativer Ausbeute erhalten.
HPLC (Methode 1): R_{T} = 14,9 min.;
HPLC (Methode 4): 80,8 Gew.%.

### Beispiel 15

### {8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester / Veresterung Racemat

(±)-(8-Fluor-2-[4-(3-methoxyphenyl)piperazin-1-yl]-3-(2-methoxy-5-trifluormethylphenyl)-3,4-dihydrochinazolin-4-yl)essigsäure (54 g) werden in Methanol (540 g) gelöst, dann wird konzentrierte Schwefelsäure (7,85 mL) zugegeben. Das Gemisch wird 26 h bei Rückfluss gerührt, dann abgekühlt und im Vakuum auf ca. ein Drittel des ursprünglichen Volumens aufkonzentriert. Wasser (150 mL) und Dichlormethan (150 mL) werden zugegeben, dann werden die Phasen getrennt. Die organische Phase wird mit gesättigter Natriumhydrogencarbonat-Lösung (zweimal 140 mL) extrahiert, über Natriumsulfat getrocknet und zu einem schaumigen Rückstand eingeengt. Dieser wird zweimal nacheinander in Ethanol (je 150 mL) gelöst und eingeengt, anschließend 18 h im Vakuum mit Schleppstickstoff getrocknet. So werden insgesamt 41,6 g *{8-Fluor-2-[4-(3-methoxyphenyl)piperazin-1-yl]-3-{2-methoxy-5-(trifluormethyl)phenyl}-3,4-dihydrochinazolin-4-yl}essigsäuremethylester* als amorpher Feststoff erhalten, entsprechend 75,2 % der Theorie.
HPLC (Methode 1): R_{T} = 16,8 min.;
HPLC (Methode 4): 85,3 Gew.%;
HPLC (Methode 3): -8,5 % e.e.

### Beispiel 16

### (2S,3S)-2,3-Bis[(4-methylbenzoyl)oxy]bernsteinsäure - {8-Fluor-1-[4-(3-methoxyphenyl)piperazin-1-yl]-3-[2-methoxy-5-(trifluormethyl)phenyl]-3,4-dihydrochinazolin-4-yl}essigsäuremethyl-ester (1:1-Salz) / Kristallisation verestertes Racemat

{8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester (41,0 g) wird in Ethylacetat (287 mL) suspendiert, dann wird (2*S*,3*S*)-2,3-Bis[(4-methylbenzoyl)oxy]bernsteinsäure (27,5 g) zugegeben. Das Gemisch wird 30 min. bei Raumtemperatur gerührt, dann wird mit (2*S*,3*S*)-2,3-Bis[(4-methylbenzoyl)oxy]bernsteinsäure - {8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester (1:1-Salz) (0,08 g) angeimpft. Die Suspension wird 16 h bei RT gerührt, anschließend auf 0-5 °C gekühlt und weitere 3 h gerührt, dann abgesaugt und mit kaltem Ethylacetat (0-10 °C, viermal 16 mL) nachgewaschen. Die Kristalle werden 18 h bei 45 °C im VTS mit Schleppstickstoff getrocknet. So werden insgesamt 25,4 g des Salzes als Feststoff erhalten, entsprechend 37,4 % der Theorie.
HPLC (Methode 1): R_{T} = 16,9 und 18,8 min.;
HPLC (Methode 4): 99,5 Gew.%;
HPLC (Methode 3): 99,3 % e.e.

### Beispiel 17

### (S)-{8-Fluor-2-[4-(3-methoxyphenyl)piperazin-1-yl]-3-(2-methoxy-5-trifluormethylphenyl)-3,4-dihydrochinazolin-4-yl}essigsäure / Verseifung Kristallisat

(2*S*,3*S*)-2,3-Bis[(4-methylbenzoyl)oxy]bernsteinsäure - {8-Fluor-2-[4-(3-methoxyphenyl)piperazin-1-yl]-3-[2-methoxy-5-(trifluormethyl)phenyl]-3,4-dihydrochinazolin-4-yl}essigsäuremethyl-ester (1:1-Salz) (25,1 g) werden in Ethylacetat (250 mL) suspendiert und solange mit gesättigter wässriger Natriumbicarbonatlösung (250 mL) geschüttelt, bis beide Phasen klar werden. Die Phasen werden getrennt, die organische Phase wird einrotiert. Der Rückstand wird in 1,4-Dioxan (250 mL) und 1N Natronlauge (77,4 mL) gelöst und 18 h bei Raumtemperatur gerührt. Anschließend wird der pH-Wert mit 1N Salzsäure (ca. 50 mL) auf 7,5 gestellt, MIBK (240 mL) zugegeben, dann der pH-Wert mit 1N Salzsäure (ca. 15 mL) auf 7,0 nachgestettt. Die Phasen werden getrennt, die organische Phase über Natriumsulfat getrocknet und eingeengt. Der Rückstand wird in Ethanol (90 mL) gelöst und eingeengt, dann nochmals in Ethanol (90 mL) gelöst und eingeengt. Der erstarrte Schaum wird 18 h bei 45 °C im VTS mit Schleppstickstoff getrocknet. So werden insgesamt 12 g *(S)-{8-Fluor-2-[4-(3-methoxyphenyl)piperazin-1-yl]-3-(2-methoxy-5-trifluormethylphenyl)-3,4-dihydrochinazolin-4-yl}essigsäure* als amorpher Feststoff erhalten, entsprechend 81,2 % der Theorie.
HPLC (Methode 1): R_{T} = 15,1 min;
HPLC (Methode 2): 97,5 % e.e.; Pd (ICP): <20 ppm.

### Alternatives Verfahren zur Racemisierung:

### Beispiel 18

### (±)-{8-Fluor-2-[4-(3-methoxyphenyl)piperazin-1-yl]-3-(2-methoxy-5-trifluormethylphenyl)-3,4-dihydrochinazolin-4-yl}essigsäure / Verseifung angereichertes R-Isomer aus der Mutterlauge nach Kristallisation

Die Mutterlauge aus einer Kristallisation von (2*S*,3*S*)-2,3-Bis[(4-methylbenzoyl)oxy]-bernsteinsäure - {8-Fluor-2-[4-(3-methoxyphenyl)piperazin-1-yl]-3-[2-methoxy-5-(trifluormethyl)-phenyl]-3,4-dihydrochinazolin-4-yl}essigsäuremethylester (1:1-Salz) im 207 g-Maßstab wird mit gesättigter wässriger Natriumbicarbonatlösung (500 mL) geschüttelt, die Phasen werden getrennt und die organische Phase wird mit halbgesättigter wässriger Natriumbicarbonatlösung (500 mL) geschüttelt. Die Phasen werden getrennt, die organische Phase über Natriumsulfat getrocknet und einrotiert. Der Rückstand wird in Ethanol (500 mL) gelöst und zu einem harten Schaum einrotiert. Dieser wird in 1,4-Dioxan (1,6 L) und 1N Natronlauge (1,04 L) gelöst und 18 h bei Raumtemperatur gerührt, dann wird Toluol (1,5 L) zugegeben und die Phasen getrennt. Die wässrige Phase wird mit Salzsäure (20 %ig, ca. 155 mL) von pH 14 auf pH 8 gestellt, dann wird MIBK (1,25 L) zugegeben und mit Salzsäure (20 %ig, ca. 25 mL) auf pH 7 nachgestellt. Die Phasen werden getrennt, die organische Phase über Natriumsulfat getrocknet und zum harten Schaum einrotiert. Dieser wird 18 h bei 45 °C im VTS mit Schleppstickstoff getrocknet. So werden insgesamt 150 g *{8-Fluor-2-[4-(3-methoxyphenyl)piperazin-1yl]-3-(2-methoxy-5-trifluormethylphenyl)-3,4-dihydrochinazolin-4-yl}essigsäure* als (R/S)-Gemisch als amorpher Feststoff erhaben.
HPLC (Methode 2): -14,6 % e.e.

### Beispiel 19

### (±)-{8-Fluor-2-[4-(3-methoxyphenyl)piperazin-1-yl]-3-(2-methoxy-5-trifluormethylphenyl)-3,4-dihydrochinazolin-4-yl}essigsäure / Racemisierung

{8-Fluor-2-[4-(3-methoxyphenyl)piperazin-1-yl]-3-(2-methoxy-5-trifluormethylphenyl)-3,4-dihydrochinazolin-4-yl}essigsäure (150 g, R/S-Gemisch mit -14,6 % e.e.) werden in Acetonitril (1,5 L) gelöst und mit Natriummethylat (30 %ig in Methanol, 97,2 mL) versetzt, dann 77 h bei Rückfluss gerührt. Nach Abkühlen auf Raumtemperatur wird das Gemisch im Vakuum auf die Hälfte aufkonzentriert, dann wird mit Salzsäure (20 %ig, ca. 80 mL) von pH 13 auf pH 7,5 gestellt, MIBK (0,6 L) zugegeben und mit Salzsäure (20 %ig, ca. 3 mL) auf pH 7 nachgestellt. Die Phasen werden getrennt, die organische Phase über Natriumsulfat getrocknet und zum harten Schaum einrotiert. Der Rückstand wird in Ethanol (500 mL) gelöst und eingeengt, dann nochmals in Ethanol (500 mL) gelöst und eingeengt, dann 18 h bei 45 °C im VTS mit Schleppstickstoff getrocknet. So werden insgesamt 148 g *(±)-{8-Fluor-2-[4-(3-methoxyphenyl)piperazin-1-yl]-3-(2-methoxy-5-trifluormethylphenyl)-3,4-dihydrochinazolin-4-yl}essigsäure* als amorpher Feststoff erhalten, entsprechend 98,7 % der Theorie.
HPLC (Methode 2): 1,5 % e.e.

### Beispiel 20

### {8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester (Veresterung)

(±)-{8-Fluor-2-[4-(3-methoxyphenyl)piperazin-1-yl]-3-(2-methoxy-5-trifluormethylphenyl)-3,4-dihydrochinazolin-4-yl}essigsäure (148 g) werden in Methanol (1480 g) gelöst, dann wird konzentrierte Schwefelsäure (21,5 mL) zugegeben. Das Gemisch wird 6 h bei Rückfluss gerührt, dann abgekühlt und im Vakuum auf ca. ein Drittel des ursprünglichen Volumens aufkonzentriert. Wasser (400 mL) und Dichlormethan (400 mL) werden zugegeben, dann werden die Phasen getrennt. Die organische Phase wird mit gesättigter Natriumhydrogencarbonat-Lösung (zweimal 375 mL, mit 300 mL Wasser verdünnt) extrahiert, über Natriumsulfat getrocknet und zu einem schaumigen Rückstand eingeengt. Dieser wird zweimal nacheinander in Ethanol (je 400 mL) gelöst und eingeengt, anschließend 18 h im Vakuum mit Schleppstickstoff getrocknet. So werden insgesamt 124 g *{8-Fluor-2-[4-(3-methoxyphenyl)piperazin-1-yl]-3-[2-methoxy-5-(trifluormethyl)-phenyl]-3,4-dihydrochinazolin-4-yl}essigsäuremethylester* als amorpher Feststoff erhalten, entsprechend 81,9 % der Theorie.
HPLC (Methode 1): R_{T} = 16,9 min.;

### Beispiel 21

### (2S,3S)-2,3-Bis[(4-methylbenzoyl)oxy]bernsteinsäure- {8-Fluor-2-[4-(3-methoxyphenyl)piperazin-1-yl]-3-[2-methoxy-5-(trifluormethyl)phenyl]-3,4-dihydrochinazolin-4-yl}essigsäuremethyl-ester (1:1-Salz) / Kristallisation verestertes Racemat

(2S,3S)-2,3-Bis[(4-methylbenzoyl)oxy]bernsteinsäure - {8-Fluor-2-[4-(3-methoxyphenyl)piperazin-1-yl]-3-[2-methoxy-5-(trifluormethyl)phenyl]-3,4-dihydrochinazolin-4-yl}essigsäuremethyl-ester (1:1-Salz) (123 g, -14,4 % e.e.) wird in Ethylacetat (861 mL) suspendiert und filtriert, dann wird (2*S*,3*S*)-2,3-Bis[(4-methylbenzoyl)oxy]bernsteinsäure (82,5 g) zugegeben. Das Gemisch 30 min. bei Raumtemperatur gerührt, dann wird mit (2*S*,3*S*)-2,3-Bis[(4-methylbenzoyl)oxy]bernsteinsäure - {8-Fluor-2-[4-(3-methoxyphenyl)piperazin- 1-yl]-3-[2-methoxy-5-(trifluormethyl)phenyl]-3,4-dihydrochinazolin-4-yl}essigsäuremethylester (1:1 -Salz) (0,24 g) angeimpft. Die Suspension wird 4 Tage bei RT gerührt, anschließend bis auf ca. 600 mL aufkonzentriert und nochmals mit (2*S*,3*S*)-2,3-Bis[(4-methylbenzoyl)oxy]bernsteinsäure - {8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester (1:1-Salz) (0,24g) angeimpft. Die Suspension wird 1 Woche bei RT gerührt, auf 0-5 °C gekühlt und weitere 3 h gerührt, dann abgesaugt und mit kaltem Ethylacetat (0-10 °C, 4 x 40 mL) nachgewaschen. Die Kristalle werden 18 h bei 45 °C im VTS mit Schleppstickstoff getrocknet. So werden insgesamt 11,8 g des Salzes als Feststoff erhalten, entsprechend 5,8 % der Theorie.

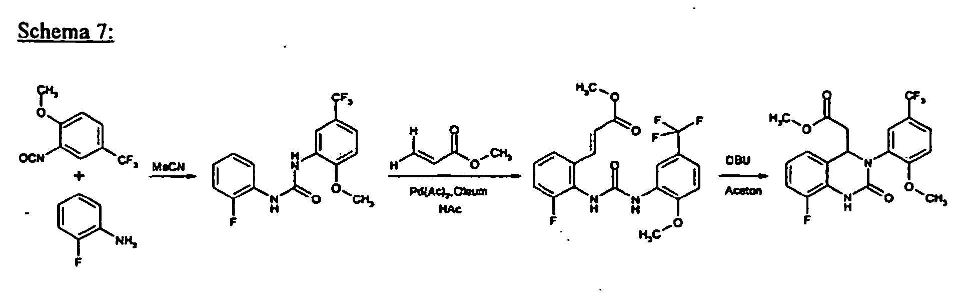

### Beispiel 22

### N-(2-Fluorphenyl)-N'-[2-methoxy-5-(trifluormethyl)phenyl]harnstoff

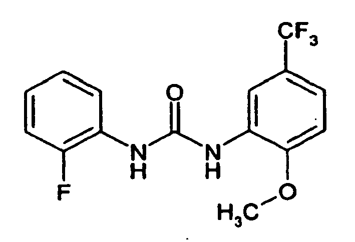

2-Methoxy-5-trifluormethylphenylisocyanat (1057,8 g) wird in Acetonitril (4240 mL) gelöst, dann wird 2-Fluoranilin (540,8 g) dazugegeben und mit Acetonitril (50 mL) nachgespült. Die resultierende klare Lösung wird 4 h bei Rückfluss (ca. 82°C) gerührt, dann bei ca. 78°C angeimpft und ca. 15 min. gerührt. Die Suspension wird auf 0°C abgekühlt, das Produkt abgesaugt und mit Acetonitril (950 mL, auf 0-5 °C gekühlt) nachgewaschen. Das Produkt wird über Nacht bei 45°C im Vakuumtrockenschrank mit Schleppstickstoff getrocknet. So werden insgesamt 1380,8 g N-*(2-fluorphenyl)*-N'-*[2-methoxy-5-(trifluormethyl)phenyl]-harnstoff* als Feststoff erhalten, entsprechend 86,4 % der Theorie.

¹H NMR (500 MHz, d₆-DMSO): δ = 9,36 (s, 1H), 9,04 (s, 1H), 8,55 (d, 1,7Hz, 1H), 8,17 (t, 8,2Hz, 1H), 7,33 (d, 8,5Hz, 1H), 7,20 - 7,26 (m, 2H), 7,14 (t, 7,6Hz, 1H), 7,02 (m, 1H), 3,97 (s, 3H) ppm;
MS (API-ES-pos.): m/z = 329 [(M+H)⁺, 100 %];
HPLC : R_{T} = 48,7 min.

Instrument: HP 1100 mit Multiple Wavelength-Detektion; Säule: Phenomenex-Prodigy ODS (3) 100A, 150 mm x 3 mm, 3 µm; Eluent A: (1,36 g KH₂PO₄ + 0,7 mL H₃PO₄) /L Wasser, Eluent B: Acetonitril; Gradient: 0 min 20% B, 40 min 45% B, 50 min 80% B, 65 min 80% B; Fluss: 0,5 mL/min; Temp.: 55 °C; UV-Detektion: 210 nm.

### Beispiel 23

### Methyl(2E)-3-{3-fluor-2-[({[2-methoxy-5-(trifluormethyl)phenyl]amino}carbonyl)amino]phenyl}-acrylat

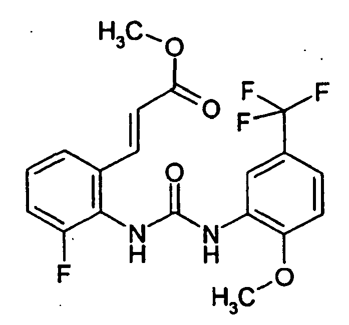

N-(2-Fluorphenyl)-N'-[2-methoxy-5-(trifluormethyl)phenyl]harnstoff (0,225 kg) wird in Essigsäure (6,75 L) gelöst und mit Palladiumacetat (30,3 g) versetzt. Dann wird 65%iges Oleum (247,5 g) zudosiert und anschließend Methylacrylat (90 g) zugegeben. Die Lösung wird über Nacht bei Raumtemperatur gerührt. Anschließend wird bei ca. 30°C und ca. 30 mbar Essigsäure (3740 g) abdestilliert. Die Suspension wird mit Wasser (2,25 L) versetzt und ca. 1 Stunde nachgerührt. Das Produkt wird abgesaugt, zweimal mit Wasser (0,5 L) gewaschen und über Nacht bei 50°C im Vakuumtrockenschrank mit Schleppstickstoff getrocknet. So werden insgesamt 210,3 g *Methyl(2E)-3-{3-fluor-2-[({[2-methoxy-5-(trifluormethyl)phenyl]amino}carbonyl)amino]phenyl}-acrylat* als Feststoff erhalten, entsprechend 72,2 % der Theorie.

¹H NMR (300 MHz, d₆-DMSO): δ = 9,16 (s, 1H), 8,84 (s, 1H), 8,45 (d, 1,7Hz, 1H), 7,73 (m, 2H), 7,33 (m, 3H), 7,22 (d, 8,6Hz, 1H), 6,70 (d, 16Hz, 1H), 3,99 (s, 3H), 3,71 (s, 3H) ppm;
MS (API-ES-pos.): m/z = 429,9 [(M+NH₄)⁺]; 412,9 [(M+H)⁺]
HPLC : R_{T} = 46,4 min.

Instrument: HP 1100 mit Multiple Wavelength-Detektion; Säule: Phenomenex-Prodigy ODS (3) 100A, 150 mm x 3 mm, 3 µm; Eluent A: (1,36 g KH₂PO₄ + 0,7 mL H₃PO₄) / L Wasser, Eluent B: Acetonitril; Gradient: 0 min 20% B, 40 min 45% B, 50 min 80% B, 65 min 80% B; Fluss: 0,5 mL/min; Temp.: 55 °C; UV-Detektion: 210 nm.

### Beispiel 24

### {8-Fluor-3-[2-methoxy-5-(trifluormethyl)phenyl]-2-oxo-1,2,3,4-tetrahydrochinazolin-4-yl}essigsäuremethylester

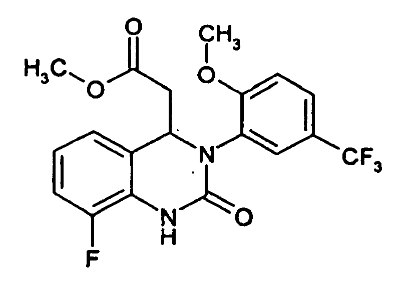

Methyl(2E)-3-{3-fluor-2-[({[2-methoxy-5-(trifluormethyl)phenyl]amino}carbonyl)amino]phenyl}-acrylat (50 g) wird in Aceton (1,2 L) suspendiert und mit 1,8-Diazobicyclo[5.4.0]undec-7-en (3,7 g) versetzt. Die Suspension wird auf Rückfluß (ca..56°C) erwärmt und für 4 h gerührt. Die entstehende blanke Lösung wird warm über Kieselgur (5 g) filtriert. Das Kieselgur wird mit warmem Aceton (100 ml) nachgespült. Anschließend wird Aceton (550 g) abdestilliert. Die entstehende Suspension wird in 3 h auf 0°C abgekühlt und nachgerührt. Das Produkt wird abgesaugt, zweimal mit kaltem Aceton (50 ml) gewaschen und über Nacht bei 45 °C im Vakuumtrockenschrank mit Schleppstickstoff getrocknet. So werden insgesamt 44,5 g *{8-Fluor-3-[2-methoxy-5-(trifluormethyl)phenyl]-2-oxo-1,2,3,4-tetrahydrochinazo-lin-4-yl}essigsäure-methylester* als Feststoff erhalten, entsprechend 89 % der Theorie.

¹H NMR (300 MHz, d₆-DMSO): δ = 9,73 (s, 1H), 7,72 (d, ²*J* = 7,3, 1H), 7,71 (s, 1H), 7,33 (d, ²*J* = 9,3, 1H), 7,15 (dd, ²*J* = 9,6, ²*J* = 8,6, 1H), 7,01 (d, ²*J* = 7,3, 1H), 6,99-6,94 (m, 1H), 5,16 (t, ²*J* = 5,9, 1H), 3,84 (s, 3H), 3,41 (s, 3H), 2,81 (dd, ²*J*= 15,4, ²*J* = 5,8, 1H), 2,62 (dd, ²*J*= 15,4, ²*J* = 6,3, 1H) ppm;
MS (API-ES-pos.): m/z = 413 [(M+H)⁺, 100 %], 825 [(2M+H)⁺, 14 %];
HPLC : R_{T} = 37,1 min.

Instrument: HP 1100 mit Multiple Wavelength-Detektion; Säule: Phenomenex-Prodigy ODS (3) 100A, 150 mm x 3 mm, 3 µm; Eluent A: (1,36 g KH₂PO₄ + 0,7 mL H₃PO₄) / L Wasser, Eluent B: Acetonitril; Gradient: 0 min 20% B, 40 min 45% B, 50 min 80% B, 65 min 80% B; Fluss: 0,5 mL/min; Temp.: 55 °C; UV-Detektion: 210 nm.

## Patentansprüche

1. Verfahren zur Herstellung einer Verbindung der Formel (I) 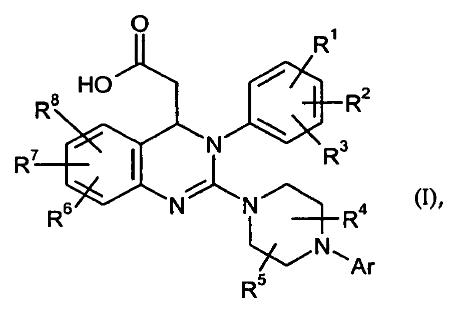 in welcher
Ar für Aryl steht, worin Aryl substituiert sein kann mit 1 bis 3 Substituenten, wobei die Substituenten unabhängig voneinander ausgewählt werden aus der Gruppe, bestehend aus Alkyl, Alkoxy, Formyl, Carboxyl, Alkylcarbonyl, Alkoxycarbonyl, Trifluormethyl, Halogen, Cyano, Hydroxy, Amino, Alkylamino, Aminocarbonyl und Nitro,
worin Alkyl substituiert sein kann mit 1 bis 3 Substituenten, wobei die Substituenten unabhängig voneinander ausgewählt werden aus der Gruppe, bestehend aus Halogen, Amino, Alkylamino, Hydroxy und Aryl,
oder zwei der Substituenten am Aryl zusammen mit den Kohlenstoffatomen, an die sie gebunden sind, ein 1,3-Dioxolan, einen Cyclopentan-Ring oder einen Cyclohexan-Ring bilden und ein gegebenenfalls
vorhandener dritter Substituent unabhängig davon aus der genannten Gruppe ausgewählt wird,
R¹ für Wasserstoff, Amino, Alkyl, Alkoxy, Alkylamino, Alkylthio, Cyano, Halogen, Nitro oder Trifluormethyl steht,
R² für Wasserstoff, Alkyl, Alkoxy, Alkylthio, Cyano, Halogen, Nitro oder Trifluormethyl steht,
R³ für Amino, Alkyl, Alkoxy, Alkylamino, Alkylthio, Cyano, Halogen, Nitro, Trifluormethyl, Alkylsulfonyl oder Alkylaminosulfonyl steht
oder
einer der Reste R¹, R² und R³ für Wasserstoff, Alkyl, Alkoxy, Cyano, Halogen, Nitro oder Trifluormethyl steht und die anderen beiden zusammen mit den Kohlenstoffatomen, an die sie gebunden sind, ein 1,3-Dioxolan, einen Cyclopentan-Ring oder einen Cyclohexan-Ring bilden,
R⁴ für Wasserstoff oder Alkyl steht,
R⁵ für Wasserstoff oder Alkyl steht
oder
die Reste R⁴ und R⁵ im Piperazin-Ring an genau gegenüberliegenden Kohlenstoffatomen gebunden sind und eine gegebenenfalls mit 1 bis 2 Methylgruppen substituierte Methylen-Brücke bilden,
R⁶ für Wasserstoff, Alkyl, Alkoxy, Alkylthio, Formyl, Carboxyl, Aminocarbonyl, Alkylcarbonyl, Alkoxycarbonyl, Trifluormethyl, Halogen, Cyano, Hydroxy oder Nitro steht,
R⁷ für Wasserstoff, Alkyl, Alkoxy, Alkylthio, Formyl, Carboxyl, Alkylcarbonyl, Alkoxycarbonyl, Trifluormethyl, Halogen, Cyano, Hydroxy oder Nitro steht
und
R⁸ für Wasserstoff, Alkyl, Alkoxy, Alkylthio, Formyl, Carboxyl, Alkylcarbonyl, Alkoxycarbonyl, Trifluormethyl, Halogen, Cyano, Hydroxy oder Nitro steht,
durch Verseifung des Esters einer Verbindung der Formel (II) 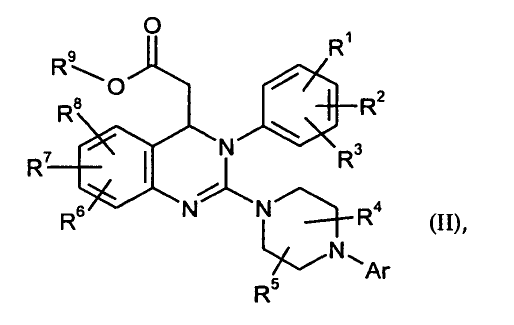 in welcher
Ar, R¹, R⁷, R³, R⁴, R⁵, R⁶, R⁷ und R⁸ die oben angegebene Bedeutung haben, und
R⁹ für C₁-C₄-Alkyl steht,
mit einer Base oder einer Säure, **dadurch gekennzeichnet, dass** die Verbindung der Formel (II) hergestellt wird durch Umsetzung einer Verbindung der Formel (III) 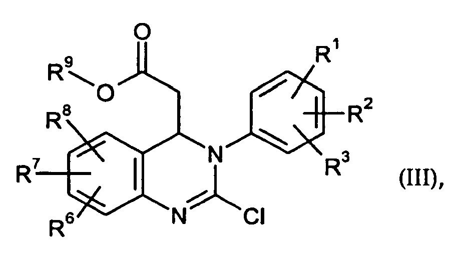 in welcher
R¹, R², R³, R⁶, R⁷ und R⁸ die oben angegebene Bedeutung haben, und
R⁹ für C₁-C₄-Alkyl steht,
in Gegenwart einer Base
mit einer Verbindung der Formel (IV) 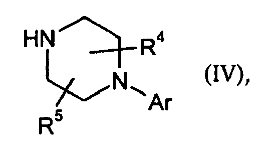 in welcher
Ar, R⁴ und R⁵ die oben angegebene Bedeutung haben.

2. Verfahren nach Anspruch 1, **dadurch gekennzeichnet, dass** die Verbindung der Formel (III), wie in Anspruch 1 definiert, hergestellt wird durch Umsetzung einer Verbindung der Formel (V) 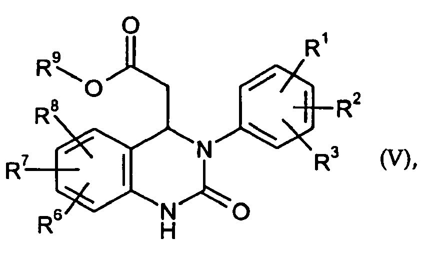 in welcher
R¹, R², R³, R⁶, R⁷ und R⁸ die in Anspruch 1 angegebene Bedeutung haben, und
R⁹ für C₁-C₄-Alkyl steht,
mit Phosphoroxychlorid, Phosphortrichlorid oder Phosphorpentachlorid, in Gegenwart einer Base.

3. Verfahren nach Anspruch 2, **dadurch gekennzeichnet, dass** die Verbindung der Formel (V), wie in Anspruch 2 definiert, hergestellt wird durch Umsetzung einer Verbindung der Formel (VI) 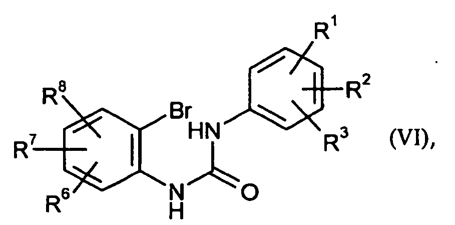 in welcher
R¹, R², R³, R⁶, R⁷ und R⁸ die in Anspruch 2 angegebene Bedeutung haben,
mit einer Verbindung der Formel 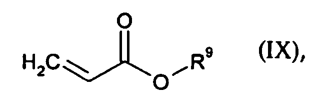 in welcher
R⁹ für C₁-C₄-Alkyl steht,
in Gegenwart eines Palladiumkatalysators und einer Base.

4. Verfahren nach Anspruch 2, **dadurch gekennzeichnet, dass** die Verbindung der Formel (V), wie in Anspruch 2 definiert, hergestellt wird durch Umsetzung einer Verbindung der Formel (VII) 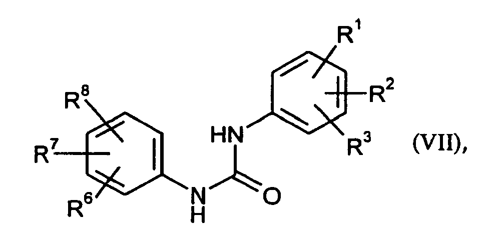 in welcher
R¹, R², R³, R⁶, R⁷ und R⁸ die in Anspruch 2 angegebene Bedeutung haben,
in der ersten Stufe mit einer Verbindung der Formel (IX) in Essigsäure in Gegenwart eines Palladiumkatalysators, eines Oxidationsmittels und einer Säure zu einer Verbindung der Formel (VIII) 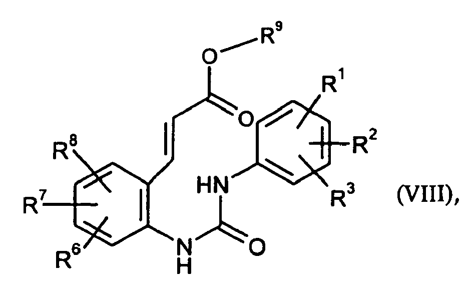 in welcher
R¹, R², R³, R⁶, R⁷ und R⁸ die in Anspruch 2 angegebene Bedeutung haben, und
R⁹ für C₁-C₄-Alkyl steht,
und in der zweiten Stufe mit einer Base zu einer Verbindung der Formel (V).

5. Verfahren nach einem der Ansprüche 1 bis 4, **dadurch gekennzeichnet, dass** die Verbindung der Formel (I) gleich
{8-Fluor-2-[4-(3-methoxyphenyl)-piperazin-1-yl]-3-[2-methoxy-5-(trifluormethyl)phenyl]-3,4-dihydrochinazolin-4-yl}essigsäure 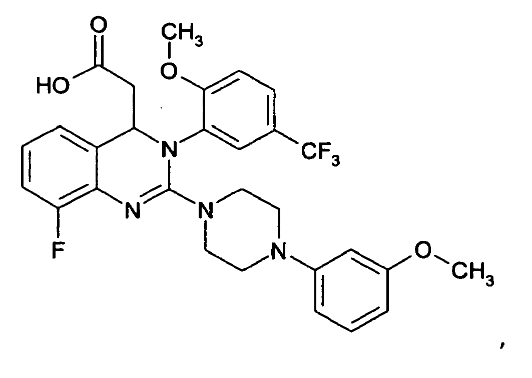 die Verbindung der Formel (II) gleich
{8-Fluor-2-[4-(3-methoxyphenyl)-piperazin-1-yl]-3-[2-methoxy-5-(trifluormethyl)phenyl]-3,4-dihydrochinazolin-4-yl}essigsäuremethylester 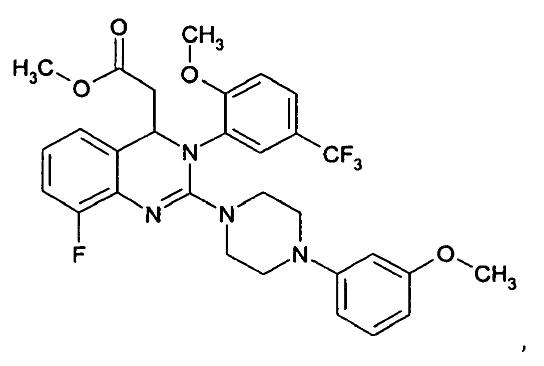 die Verbindung der Formel (III) gleich
2-Chlor-8-fluor-3-[2-methoxy-5-(trifluormethyl)phenyl]-3,4-dihydrochinazolin-4-yl}essigsäuremethylester 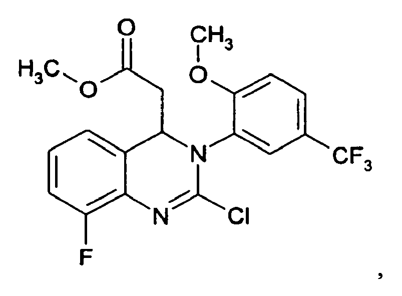 die Verbindung der Formel (IV) gleich
1-(3-Methoxyphenyl)piperazin 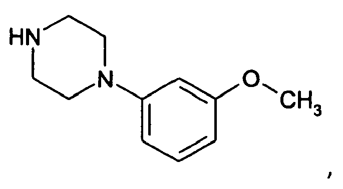 die Verbindung der Formel (V) gleich
{8-Fluor-3-[2-methoxy-5-(trifluormethyl)phenyl]-2-oxo-1,2,3,4-tetrahydrochinazolin-4-yl}essigsäuremethylester 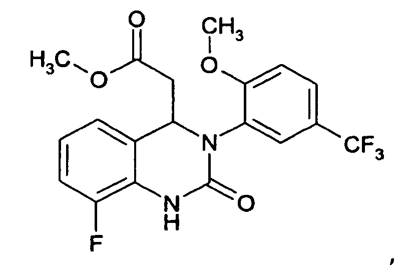 die Verbindung der Formel (VI) gleich
N-(2-Brom-6-fluorphenyl)-N'-[2-methoxy-5-(trifluormethyl)phenyl]harnstoff 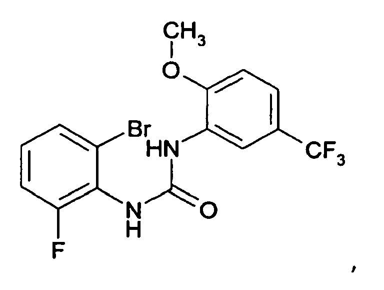 die Verbindung der Formel (VII) gleich *N*-(2-Fluorphenyl)-*N*'-[2-methoxy-5-(trifluormethyl)phenyl]harnstoff 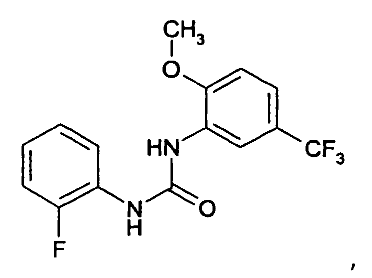 die Verbindung der Formel (VIII) gleich
Methyl(2E)-3-{3-fluor-2-[({[2-methoxy-5-(trifluormethyl)phenyl]amino} carbonyl)-amino]phenyl}-acrylat 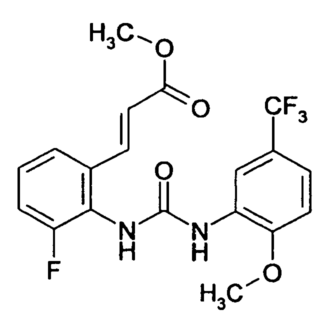 und
die Verbindung der Formel (IX) gleich
Acrylsäuremethylester 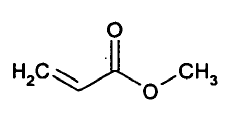 ist.

6. Verfahren zur Enantiomerentrennung von {8-Fluor-2-[4-(3-methoxyphenyl)-piperazin-1-yl]-3-[2-methoxy-5-(trifluormethyl)phenyl]-3,4-di-ydrochinazolin-4-yl}essigsäure-(C₁-C₄)-alkylester und Isolierung von (S)-{8-Fluor-2-[4-(3-methoxyphenyl)-piperazin-1-yl]-3-[2-methoxy-5-(trifluormethyl)phenyl]-3,4-di-hydrochinazolin-4-yl}essigsäure-(C₁-C₄)-alkylester, **dadurch gekennzeichnet, dass** der racemische Ester mit (2*S*,3*S*)-2,3-Bis[(4-methylbenzoyl)oxy]-bernsteinsäure kristallisiert wird.

7. (S)-{8-Fluor-2-[4-(3-methoxyphenyl)-piperazin-1-yl]-3-[2-methoxy-5-trifluormethyl)-phenyl]-3,4-di-hydrochinazolin-4-yl}essigsäuremethylester - (2*S*,3*S*)-2,3-Bis[(4-methyl-benzoyl)oxy]bernsteinsäure-Salz.

8. Verbindung der Formel (III) 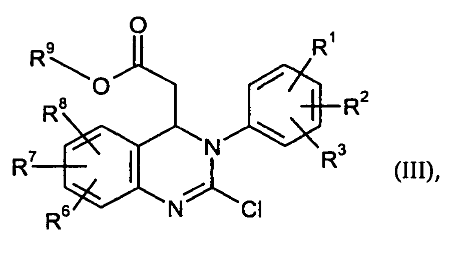 in welcher
R¹, R², R³, R⁶, R⁷ und R⁸ die in Anspruch 1 angegebene Bedeutung haben, und
R⁹ für C₁-C₄-Alkyl steht.

9. Verbindung nach Anspruch 8, **dadurch gekennzeichnet, dass** es sich bei der Verbindung um 2-Chlor-8-fluor-3-[2-methoxy-5-(trifluormethyl)phenyl]-3,4-dihydrochinazolin-4-yl}-essigsäuremethylester 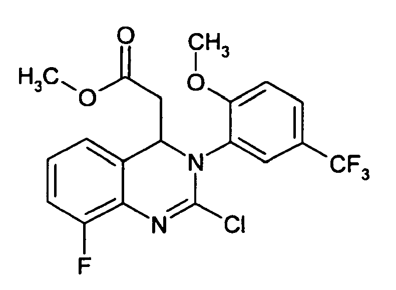 handelt.

10. Verbindung der Formel (V) 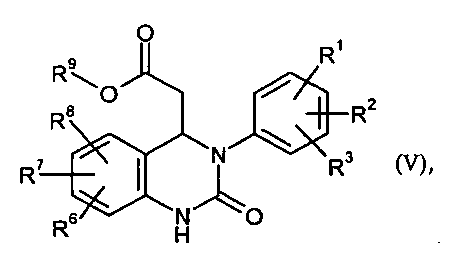 in welcher
R¹, R², R³, R⁶, R⁷ und R⁸ die in Anspruch 1 angegebene Bedeutung haben,
R⁶ für Alkyl, Alkoxy, Alkylthio, Formyl, Carboxyl, Aminocarbonyl, Alkylcarbonyl, Alkoxycarbonyl, Trifluormethyl, Halogen, Cyano, Hydroxy oder Nitro steht,
und
R⁹ für C₁-C₄-Alkyl steht.

11. Verbindung nach Anspruch 10, **dadurch gekennzeichnet, dass** es sich bei der Verbindung um {8-Fluor-3-[2-methoxy-5-(trifluormethyl)phenyl]-2-oxo-1,2,3,4-tetrahydrochinazolin-4-yl}essigsäuremethylester 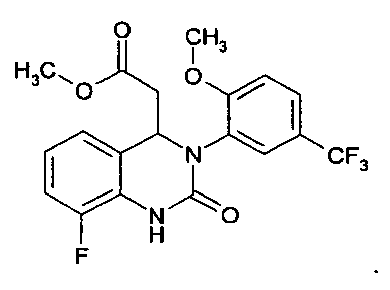 handelt.

## Claims

1. Method for the preparation of a compound of formula (I) 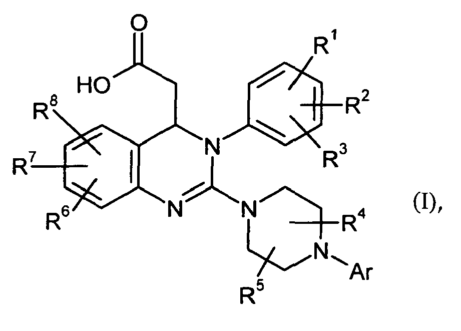 in which
Ar represents aryl, wherein aryl may be substituted with 1 to 3 substituents, whereby the substituents are selected independently of one another from the group consisting of alkyl, alkoxy, formyl, carboxy, alkylcarbonyl, alkoxycarbonyl, trifluoromethyl, halogen, cyano, hydroxy, amino, alkylamino, aminocarbonyl and nitro,
wherein alkyl may be substituted with 1 to 3 substituents, whereby the substituents are selected independently of one another from the group consisting of halogen, amino, alkylamino, hydroxy and aryl,
or two of the substituents on the aryl, together with the carbon atoms to which they are bonded, form a 1,3-dioxolane, a cyclopentane ring or a cyclohexane ring and an optionally present third substituent is selected independently thereof from the group mentioned,
R¹ represents hydrogen, amino, alkyl, alkoxy, alkylamino, alkylthio, cyano, halogen, nitro or trifluoromethyl,
R² represents hydrogen, alkyl, alkoxy, alkylthio, cyano, halogen, nitro or trifluoromethyl,
R³ represents amino, alkyl, alkoxy, alkylamino, alkylthio, cyano, halogen, nitro, trifluoromethyl, alkylsulfonyl or alkylaminosulfonyl
or
one of the radicals R¹, R² and R³ represents hydrogen, alkyl, alkoxy, cyano, halogen, nitro or trifluoromethyl and the other two, together with the carbon atoms to which they are bonded, form a 1,3-dioxolane, a cyclopentane ring or a cyclohexane ring,
R⁴ represents hydrogen or alkyl,
R⁵ represents hydrogen or alkyl
or
the radicals R⁴ and R⁵ in the piperazine ring are bonded to carbon atoms directly opposing each other and form a methylene bridge which is optionally substituted with 1 to 2 methyl groups,
R⁶ represents hydrogen, alkyl, alkoxy, alkylthio, formyl, carboxy, aminocarbonyl, alkylcarbonyl, alkoxycarbonyl, trifluoromethyl, halogen, cyano, hydroxy or nitro,
R⁷ represents hydrogen, alkyl, alkoxy, alkylthio, formyl, carboxy, alkylcarbonyl, alkoxycarbonyl, trifluoromethyl, halogen, cyano, hydroxy or nitro
and
R⁸ represents hydrogen, alkyl, alkoxy, alkylthio, formyl, carboxy, alkylcarbonyl, alkoxycarbonyl, trifluoromethyl, halogen, cyano, hydroxy or nitro,
by hydrolysis of the ester of a compound of formula (II) 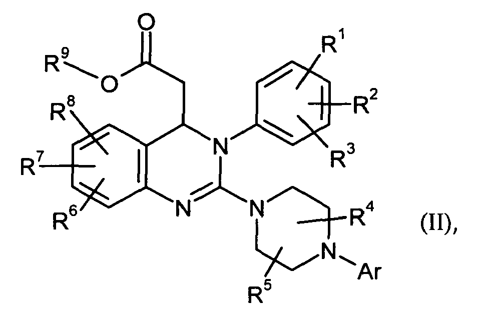 in which
Ar, R¹, R², R³, R⁴, R⁵, R⁶, R⁷ and R⁸ have the meaning indicated above, and
R⁹ represents C₁-C₄-alkyl,
using a base or an acid, **characterized in that** the compound of formula (II) is prepared by the reaction of a compound of formula (III) 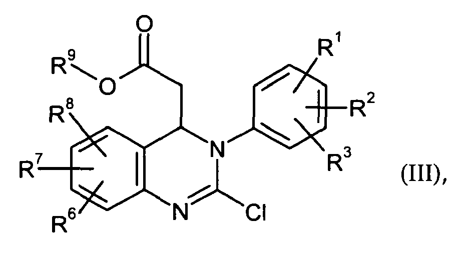 in which
R¹, R², R³, R⁶, R⁷ and R⁸ have the meaning indicated above, and
R⁹ represents C₁-C₄-alkyl,
in the presence of a base
with a compound of formula (IV) 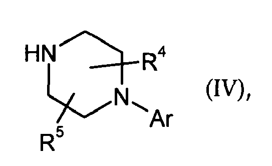 in which
Ar, R⁴ and R⁵ have the meaning indicated above.

2. Method according to claim 1, **characterized in that** the compound of formula (III), as defined in Claim 1, is prepared by the reaction of a compound of formula (V) 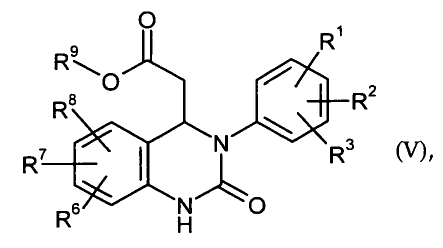 in which
R¹, R², R³, R⁶, R⁷ and R⁸ have the meaning indicated in Claim 1, and
R⁹ represents C₁-C₄-alkyl,
with phosphorus oxychloride, phosphorus trichloride or phosphorus pentachloride, in the presence of a base.

3. Method according to claim 2, **characterized in that** the compound of formula (V), as defined in Claim 2, is prepared by the reaction of a compound of formula (VI) 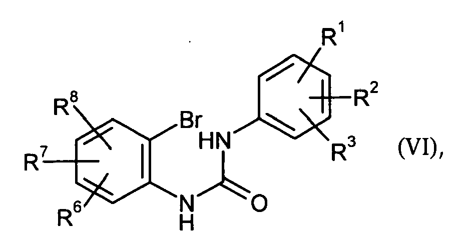 in which
R¹, R², R³, R⁶, R⁷ and R⁸ have the meaning indicated in Claim 2,
with a compound of formula 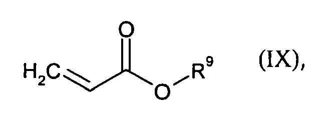 in which
R⁹ represents C₁-C₄-alkyl,
in the presence of a palladium catalyst and a base.

4. Method according to claim 2, **characterized in that** the compound of formula (V), as defined in Claim 2, is prepared by the reaction of a compound of formula (VII) 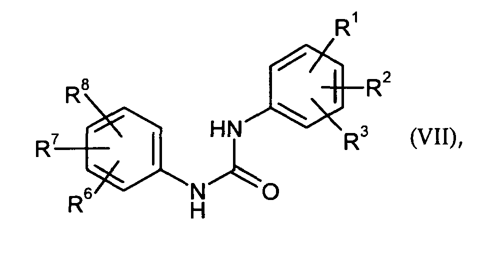 in which
R¹, R², R³, R⁶, R⁷ and R⁸ have the meaning indicated in Claim 2,
in the first stage with a compound of formula (IX) in acetic acid in the presence of a palladium catalyst, an oxidizing agent and an acid to give a compound of formula (VIII) 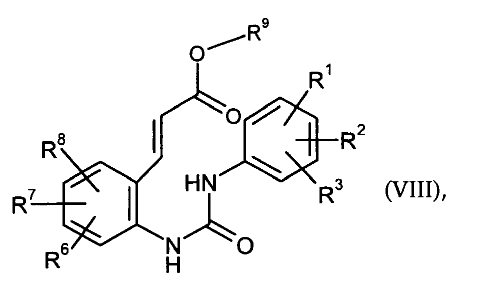 in which
R¹, R², R³, R⁶, R⁷ and R⁸ have the meaning indicated in Claim 2, and
R⁹ represents C₁-C₄-alkyl,
and in the second stage with a base to give a compound of formula (V).

5. Method according to one of claims 1 to 4, **characterized in that** the compound of formula (I) is
{8-fluoro-2-[4-(3-methoxyphenyl)piperazin-1-yl]-3-[2-methoxy-5-(trifluoromethyl)phenyl]-3,4-dihydroquinazolin-4-yl}acetic acid 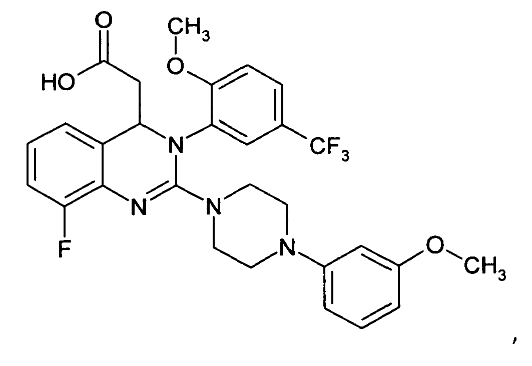 the compound of formula (II) is
methyl {8-fluoro-2-[4-(3-methoxyphenyl)piperazin-1-yl]-3-[2-methoxy-5-(trifluoromethyl)phenyl]-3,4-dihydroquinazolin-4-yl}acetate 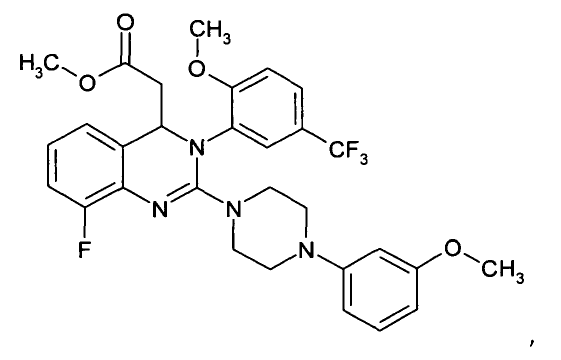 the compound of formula (III) is
methyl 2-chloro-8-fluoro-3-[2-methoxy-5-(trifluoromethyl)phenyl]-3,4-dihydroquinazolin-4-yl}acetate 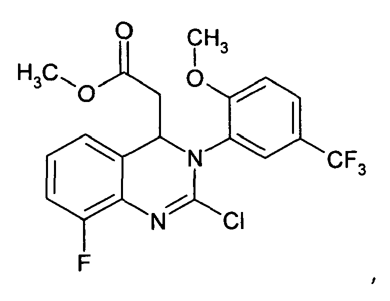 the compound of formula (IV) is
1-(3-methoxyphenyl)piperazine 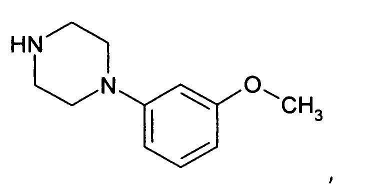 the compound of formula (V) is
methyl {8-fluoro-3-[2-methoxy-5-(trifluoromethyl)phenyl]-2-oxo-1,2,3,4-tetrahydroquinazolin-4-yl}acetate 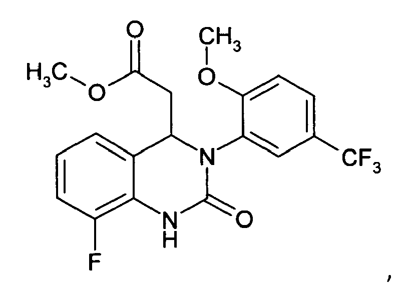 the compound of formula (VI) is
*N*-(2-bromo-6-fluorophenyl)-*N*'-[2-methoxy-5-(trifluoromethyl)phenyl]urea 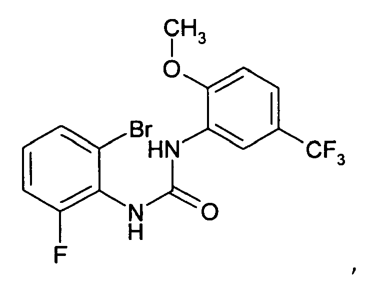 the compound of formula (VII) is
*N*-(2-fluorophenyl)-*N*'-[2-methoxy-5-(trifluoromethyl)phenyl]urea 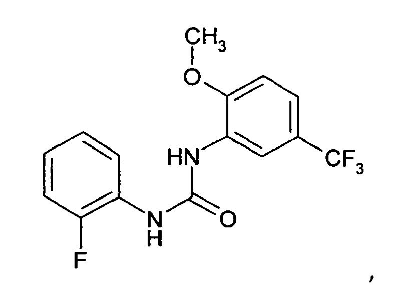 the compound of formula (VIII) is
methyl (2E)3-{3-fluoro-2-[({[2-methoxy-5-(trifluoromethyl)phenyl]amino}carbonyl)amino]phenyl}acrylate 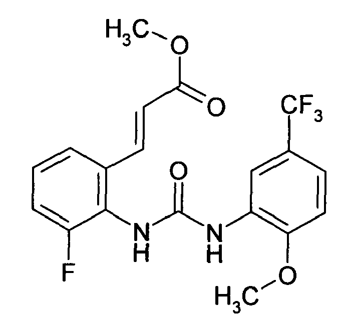 and
the compound of formula (IX) is
methyl acrylate

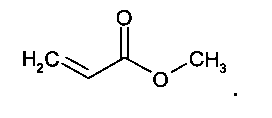

6. Method for the separation of the enantiomers of (C₁-C₄)-alkyl {8-fluoro-2-[4-(3-methoxyphenyl)piperazin-1-yl]-3-[2-methoxy-5-(trifluoromethyl)phenyl]-3,4-dihydroquinazolin-4-yl}acetate and the isolation of (C₁-C₄)-alkyl (S)-{8-fluoro-2-[4-(3-methoxyphenyl)piperazin-1-yl]-3-[2-methoxy-5-(trifluoromethyl)phenyl]-3,4-dihydroquinazolin-4-yl}acetate, **characterized in that** the racemic ester is crystallized with (2*S*,3*S*)-2,3-bis[(4-methylbenzoyl)oxy]succinic acid.

7. Methyl (*S*)-{8-fluoro-2-[4-(3-methoxyphenyl)piperazin-1-yl]-3-[2-methoxy-5-(trifluoromethyl)phenyl]-3,4-dihydroquinazolin-4-yl}acetate (2*S*,3*S*)-2,3-bis[(4-methylbenzoyl)oxy]succinic acid salt.

8. Compound of formula (III) 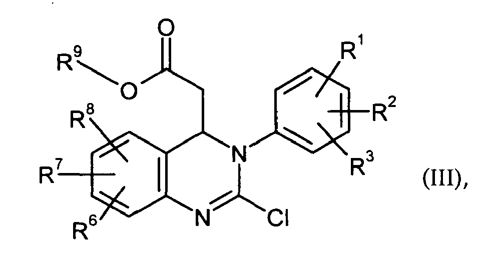 in which
R¹, R², R³, R⁶, R⁷ and R⁸ have the meaning indicated in Claim 1, and
R⁹ represents C₁-C₄-alkyl.

9. Compound according to Claim 8, **characterized in that** the compound is methyl 2-chloro-8-fluoro-3-[2-methoxy-5-(trifluoromethyl)phenyl]-3,4-dihydroquinazolin-4-yl}acetate

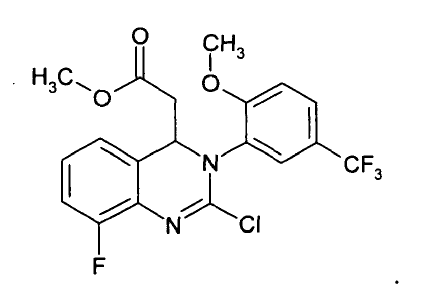

10. Compound of formula (V) 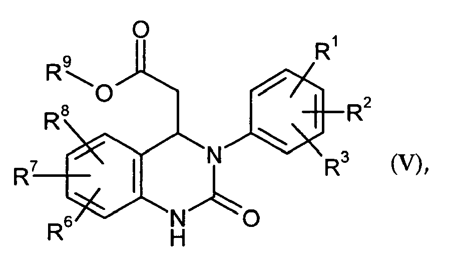 in which
R¹, R², R³, R⁷ and R⁸ have the meaning indicated in Claim 1,
R⁶ represents alkyl, alkoxy, alkylthio, formyl, carboxy, aminocarbonyl, alkylcarbonyl, alkoxycarbonyl, trifluoromethyl, halogen, cyano, hydroxy or nitro,
and
R⁹ represents C₁-C₄-alkyl.

11. Compound according to Claim 10, **characterized in that** the compound is methyl {8-fluoro-3-[2-methoxy-5-(trifluoromethyl)phenyl]-2-oxo-1,2,3,4-tetrahydroquinazolin-4-yl}acetate

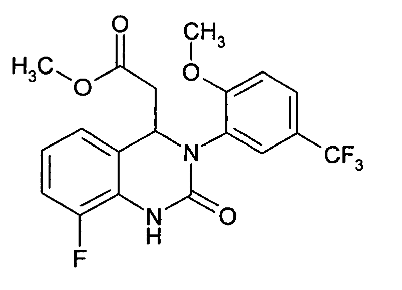

## Revendications

1. Procédé de production d'un composé de formule (I) 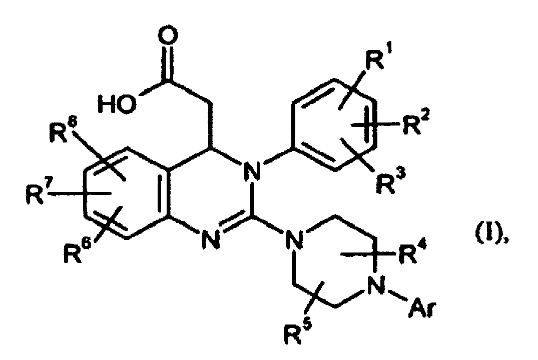 dans laquelle
Ar est un groupe aryle, le groupe aryle pouvant être substitué par 1 à 3 substituants, les substituants étant choisis indépendamment les uns des autres dans le groupe comprenant les groupes alkyle, alcoxy, formyle, carboxyle, alkylcarbonyle, alcoxycarbonyle, trifluorométhyle, un atome d'halogène, un groupe cyano, hydroxy, amino, alkylamino, aminocarbonyle et nitro,
le groupe alkyle pouvant être substitué par 1 à 3 substituants, les substituants étant choisis indépendamment les uns des autres dans le groupe comprenant un atome d'halogène, un groupe amino, alkylamino, hydroxy et aryle,
ou deux des substituants formant sur le groupe aryle, conjointement avec les atomes de carbone auxquels ils sont liés, un groupe 1,3-dioxolane, un cycle cyclopentane ou un cycle cyclohexane et un troisième substituant éventuellement présent étant choisi indépendamment de ceux-ci dans le groupe cité,
R¹ désigne un atome d'hydrogène, un groupe amino, alkyle, alcoxy, alkylamino, alkylthio, cyano, un atome d'halogène, un groupe nitro ou trifluorométhyle,
R² désigne un atome d'hydrogène, un groupe alkyle, alcoxy, alkylthio, cyano, un atome d'halogène, un groupe nitro ou trifluorométhyle,
R³ désigne un groupe amino, alkyle, alcoxy, alkylamino, alkylthio, cyano, un atome d'halogène, un groupe nitro, trifluorométhyle, alkylsulfonyle ou alkylaminosulfonyle,
ou
l'un des radicaux R¹, R² et R³ désigne un atome d'hydrogène, un groupe alkyle, alcoxy, cyano, un atome d'halogène, un groupe nitro ou trifluorométhyle et les autres forment conjointement avec les atomes de carbone auxquels ils sont liés, un groupe 1,3-dioxolane, un cycle cyclopentane ou un cycle cyclohexane,
R⁴ désigne un atome d'hydrogène ou un groupe alkyle,
R⁵ désigne un atome d'hydrogène ou un groupe alkyle
ou
les radicaux R⁴ et R⁵ dans le cycle pipérazine sont liés aux atomes de carbone exactement opposés et forment un pont méthylène, éventuellement substitués avec 1 à 2 groupes méthyle,
R⁶ désigne un atome d'hydrogène, un groupe alkyle, alcoxy, alkylthio, formyle, carboxyle, aminocarbonyle, alkylcarbonyle, alcoxycarbonyle, trifluorométhyle, un atome d'halogène, un groupe cyano, hydroxy ou nitro,
R⁷ désigne un atome d'hydrogène, un groupe alkyle, alcoxy, alkylthio, formyle, carboxyle, alkylcarbonyle, alcoxycarbonyle, trifluorométhyle, un atome d'halogène, un groupe cyano, hydroxy ou nitro,
et
R⁸ désigne un atome d'hydrogène, un groupe alkyle, alcoxy, alkylthio, formyle, carboxyle, alkylcarbonyle, alcoxycarbonyle, trifluorométhyle, un atome d'halogène, un groupe cyano, hydroxy ou nitro,
par saponification de l'ester d'un composé de formule (II) 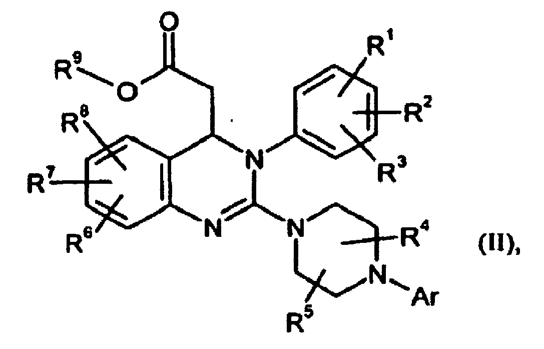 dans laquelle
Ar, R¹, R², R³, R⁴, R⁵, R⁶, R⁷ et R⁸ ont les significations indiquées ci-dessus, et
R⁹ désigne un groupe alkyle en C₁ à C₄
avec une base ou un acide, **caractérisé en ce que** le composé de formule (II) est produit par réaction d'un composé de formule (III) 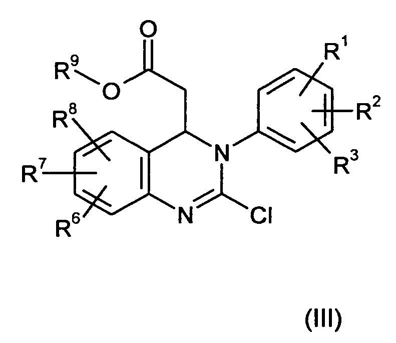 dans laquelle
R¹, R², R³, R⁶, R⁷ et R⁸ ont la signification indiquée ci-dessus, et
R⁹ désigne un groupe alkyle en C₁ à C₄
en présence d'une base
avec un composé de formule (IV) 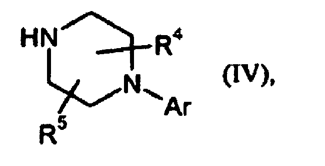 dans laquelle
Ar, R⁴ et R⁵ ont la signification indiquée ci-dessus.

2. Procédé selon la revendication 1, **caractérisé en ce que** le composé de formule (III) tel que défini dans la revendication 1, est produit par réaction d'un composé de formule (V) 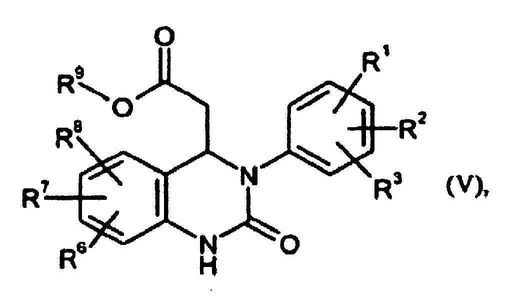 dans laquelle
R¹, R², R³, R⁶, R⁷ et R⁸ ont la signification indiquée dans la revendication 1, et
R⁹ désigne un groupe alkyle en C₁ à C₄,
avec du phophoroxychlorure, du trichlorure de phosphore ou du pentachlorure de phosphore en présence d'une base.

3. Procédé selon la revendication 2, **caractérisé en ce que** le composé de formule (V) tel que défini dans la revendication 2, est produit par réaction d'un composé de formule (VI) 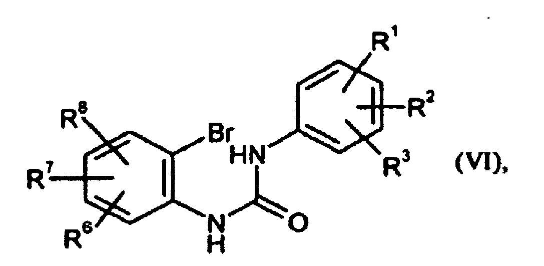 dans laquelle
R¹, R², R³, R⁶, R⁷ et R⁸ ont la signification indiquée dans la revendication 2,
avec un composé de formule 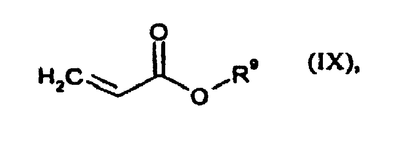 dans laquelle
R⁹ désigne un groupe alkyle en C₁ à C₄,
en présence d'un catalyseur palladium et d'une base.

4. Procédé selon la revendication 2, **caractérisé en ce que** le composé de formule (V) tel que défini dans la revendication 2, est produit par réaction d'un composé de formule (VII) 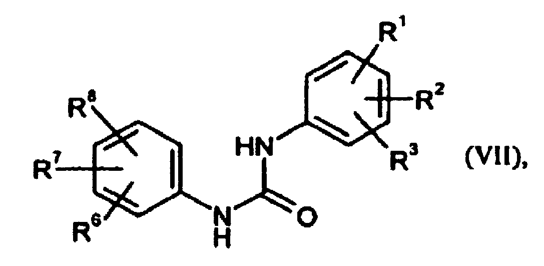 dans laquelle
R¹, R², R³, R⁶, R⁷ et R⁸ ont la signification indiquée dans la revendication 2,
à la première étape avec un composé de formule (IX) dans de l'acide acétique en présence d'un catalyseur palladium, d'un agent oxydant et d'un acide, en un composé de formule (VIII) 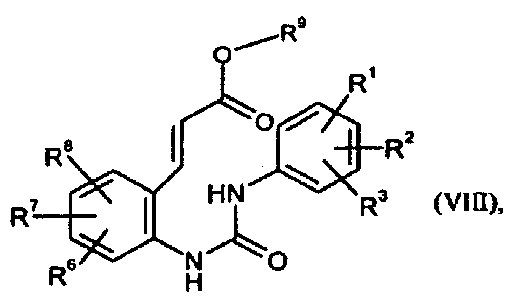 dans laquelle
R¹, R², R³, R⁶, R⁷ et R⁸ ont la signification indiquée dans la revendication 2, et
R⁹ désigne un groupe alkyle en C₁ à C₄,
et à la deuxième étape, avec une base en un composé de formule (V).

5. Procédé selon l'une des revendications 1 à 4, **caractérisé en ce que** le composé de formule (I) est
l'acide {8-fluoro-2-[4-(3-méthoxyphényl)-pipérazin-1-yl]-3-[2-méthoxy-5-(trifluorométhyl)phényl]-3,4-dihydroquinazolin-4-yl}acétique 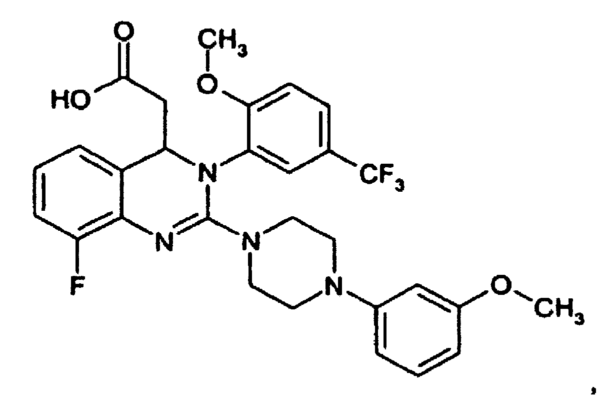 le composé de formule (II) est
l'acide {8-fluoro-2-[4-(3-méthoxyphényl)-pipérazin-1-yl]-3-[2-méthoxy-5-(trifluorométhyl)phényl]-3,4-dihydroquinazolin-4-yl}acétique méthylester 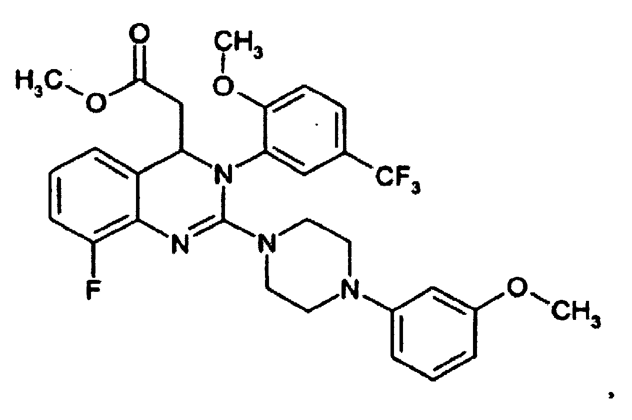 le composé de formule (III) est
l'acide 2-chloro-8-fluoro-3-[2-méthoxy-5-(trifluorométhyl)phényl]-3,4-dihydroquinazolin-4-yl}acétique méthylester 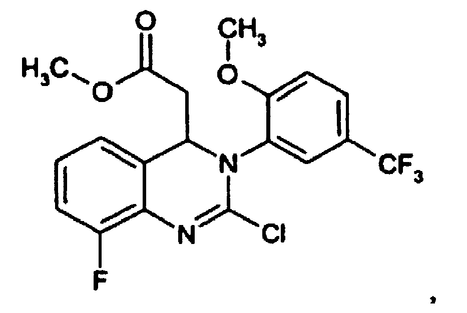 le composé de formule (IV) est
la 1-(3-méthoxyphényl)pipérazine 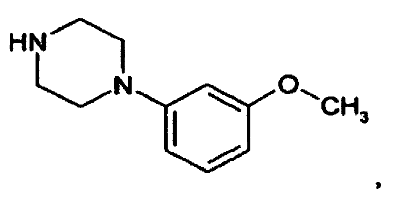 le composé de formule (V) est
l'acide {8-fluoro-3-[2-méthoxy-5-(trifluorométhyl)phényl]-2-oxo-1,2,3,4-tétrahydroquinazolin-4-yl}acétique méthylester, 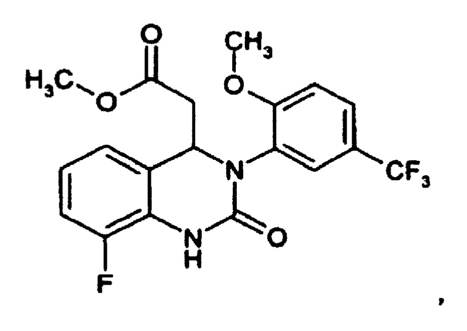 le composé de formule (VI) est
la N-(2-bromo-6-fluorophényl)-N'-[2-méthoxy-5-(trifluorométhyl)phényl] urée, 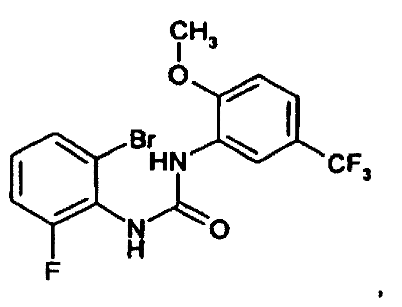 le composé de formule (VII) est
la N-(2-fluorophényl)-N'-[2-méthoxy-5-(trifluorométhyl)phényl] urée, 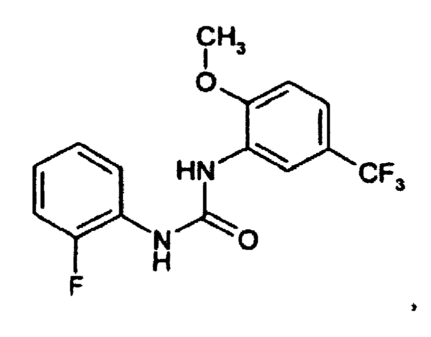 le composé de formule (VIII) est
le méthyl(2E)-3-{3-fluoro-2-[({[2-méthoxy-5-(trifluorométhyl)phényl]amino} carbonyl)amino]phényl}acrylate 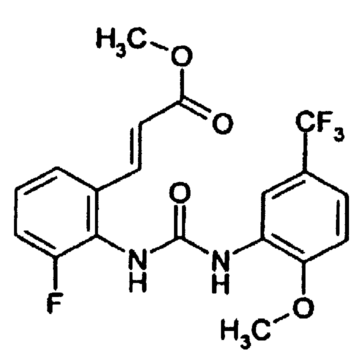 et
le composé de formule (IX) est
l'acide acrylique méthylester

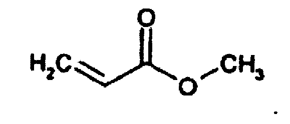

6. Procédé de séparation des énantiomères de l'acide {8-fluoro-2-[4-(3-méthoxyphényl)-pipérazin-1-yl]-3-[2-méthoxy-5-(trifluorométhyl)phényl]-3,4-dihydroquinazolin-4-yl}acétique alkylester (en C₁ à C₄) et isolement de l'acide (S)-{8-fluoro-2-[4-(3-méthoxyphényl)-pipérazin-1-yl]-3-[2-méthoxy-5-(trifluorométhyl)phényl]-3,4-dihydroquinazolin-4-yl}acétique alkylester (en C₁ à C₄), **caractérisé en ce que** l'ester racémique est cristallisé avec de l'acide (2S, 3S)-2,3-bis[(4-méthylbenzoyl)oxy}-succinique.

7. Acide (S)-{8-fluoro-2-[4-(3-méthoxyphényl)-pipérazin-1-yl]-3-[2-méthoxy-5-(trifluorométhyl)phényl]-3,4-dihydroquinazolin-4-yl}acétique méthylester - sel d'acide (2S, 3S)-2,3-bis[(4-méthyl-benzoyl)oxy]succinique.

8. Composé de formule (III) 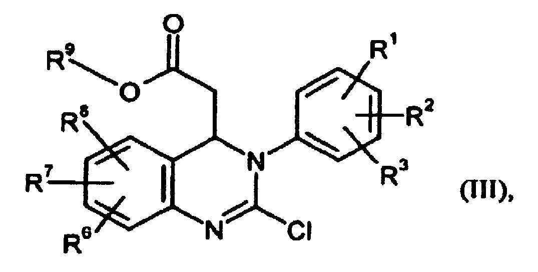 dans laquelle
R¹, R², R³, R⁶, R⁷ et R⁸ ont la signification indiquée dans la revendication 1, et
R⁹ désigne un groupe alkyle en C₁ à C₄.

9. Composé selon la revendication 8, **caractérisé en ce que** le composé est l'acide 2-chloro-8-fluoro-3-[2-méthoxy-5-(trifluorométhyl)phényl]-3,4-dihydroquinazolin-4-yl}acétique méthylester

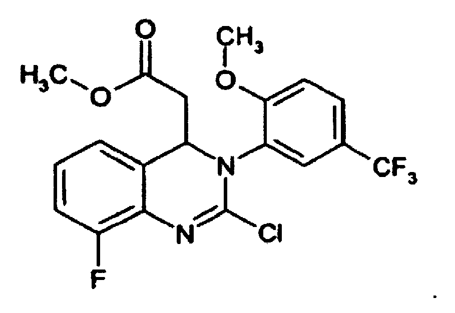

10. Composé de formule (V) 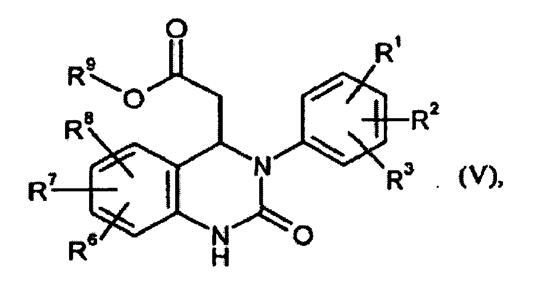 dans laquelle
R¹, R², R³, R⁷ et R⁸ ont la signification indiquée dans la revendication 1, et
R⁶ désigne un groupe alkyle, alcoxy, alkylthio, formyle, carboxyle, aminocarbonyle, alkylcarbonyle, alcoxycarbonyle, trifluorométhyle, un atome d'halogène, un groupe cyano, hydroxy ou nitro,
et
R⁹ désigne un groupe alkyle en C₁ à C₄.

11. Composé selon la revendication 10, **caractérisé en ce que** le composé est l'acide {8-fluoro-3-[2-méthoxy-5-(trifluorométhyl)phényl]-2-oxo-1,2,3,4-tétrahydroquinazolin-4-yl}acétique méthylester
